# Europäisches Patentamt

# European Patent Office

# Office européen des brevets

(11) Publication number: **0 339 302 B1**

## EUROPEAN PATENT SPECIFICATION

(45) Date of publication of patent specification: **22.12.93**  (51) Int. Cl.5: **G01N 33/86**, G01N 33/577, G01N 33/53, G01N 33/543

(21) Application number: **89105814.1**

(22) Date of filing: **03.04.89**

(54) Reagent system for immunologically assaying complex of human plasminogen activator inhibitor and human tissue plasminogen activator, and assay kit therefor.

(30) Priority: **04.04.88 JP 81366/88**
**04.04.88 JP 81367/88**
**02.12.88 JP 304217/88**
**27.12.88 JP 327998/88**
**09.01.89 JP 1256/89**

(43) Date of publication of application:
**02.11.89 Bulletin 89/44**

(45) Publication of the grant of the patent:
**22.12.93 Bulletin 93/51**

(84) Designated Contracting States:
**BE CH DE FR GB LI SE**

(56) References cited:
**WO-A-87/00549**
**WO-A-87/06346**

**CHEMICAL ABSTRACTS, vol. 109, 1988, Columbus, OH (US); J. AMIRAL et al., p. 345, no. 225319e&NUM;**

**CHEMICAL ABSTRACTS, vol. 105, 1986, Columbus, OH (US); M. PHILIPS et al., p. 263, no. 2689s&NUM;**

(73) Proprietor: **TEIJIN LIMITED**
**6-7, Minamihonmachi 1-chome**
**Chuo-ku**
**Osaka-shi Osaka 541(JP)**

(72) Inventor: **Noro, Atsushi**
**3-5-18, Tamadaira**
**Hino-shi Tokyo(JP)**
Inventor: **Ichikawa, Yataro**
**2-11-7, Kotesashi-cho**
**Tokorozawa-shi Saitama-ken(JP)**
Inventor: **Sakata, Yoichi**
**1-13-39, Kanagaki-cho**
**Oyama-shi Tochigi-ken(JP)**
Inventor: **Itoh, Kazuhiko**
**2-1-3, Yamate-cho**
**Iwakuni-shi Yamaguchi-ken(JP)**
Inventor: **Hasegawa, Ryoichi**
**1-28-5-302, Ozu-cho**
**Iwakuni-shi Yamaguchi-ken(JP)**

(74) Representative: **Kraus, Walter, Dr. et al**
**Patentanwälte Kraus, Weisert & Partner**
**Thomas-Wimmer-Ring 15**
**D-80539 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 104, 1986, Columbus, OH (US); C. KORNINGER et al., p. 251, no. 182106k&NUM;

THROMBOSIS AND HAEMOSTASIS, vol. 55, no. 3, 1986, F.K. Schattauer Verlag GmbH, Stuttgart (DE); G. URDEN et al., pp. 383-387&NUM;

CHEMICAL ABSTRACTS, vol. 105, 1986, Columbus, OH (US); L.S. NIELSEN et al., p. 263, no. 2688r&NUM;

# EP 0 339 302 B1

**Description**

This invention relates to a reagent system for immunologically assaying a complex of a human plasminogen activator inhibitor and a human tissue plasminogen activator in a human assay sample, an assay kit therefor, a method of assaying it, monoclonal antibodies therefor, and the use of these antibodies.

In the present invention, the following abbreviations are used.

PAI.1

Plasminogen activator inhibitor 1

tPA

Tissue plasminogen activator

PAI.1-tPA complex

A complex of human plasminogen activator inhibitor 1 with human tissue plasminogen activator

MCA

Monoclonal antibody

PAI.1-HMCA

A monoclonal antibody against human plasminogen activator inhibitor 1

tPA-MCA

A monoclonal antibody against human tissue plasminogen activator

tPA is an enzyme which activates plasminogen in a fibrinolytic system. The use of tPA as a fibrinolytic agent and its bioengineering production have been developed.

It is known that human plasminogen activator inhibitor 1 (human PAI.1), urokinase inhibitor (PAI.2) and urokinase inhibitor-like factor (PAI.3) are known to exist as factors which inhibit the activity of human plasminogen activator.

It is known that PAI.1 is a kind of serine protease inhibitor family and rapidly reacts with human tPA to form a complex and thus to inhibit the fibrinolytic activity of tPA [see Levin, E. G., Proc. Natl. Acad. Sci., USA, 80, 6804-6808 (1986); Philips M. et al., Biochem. Biophs. Acta., 802, 99-110 (1984)]. PAI.1-tPA complex is also known to exist in human plasma [see Booth, N. A. et al., Thromb. Res., 38, 261-267 (1985)]. Furthermore, the correlation between the PAI activity value in the blood and thrombosis was suggested [see Nilsson et al., Brit. Med. J., 290, 1453-1456 (1985), Paremo, J. A. et al., Thromb. Haemost., 54, 713-716 (1985)]. It has thus been made clear that human PAI.1 is an important control factor in an initial stage in the formation of plasmin from plasminogen in a blood fibrinolytic system.

The relation of the PAI.1-tPA complex in blood to thrombosis is also known [see Amiral J. et al., Thromb. Res., supplement VII, 99-113 (1988)].

In Thromb. Res. 1988, (Suppl. 8), 99-113 two ELISA methods using monoclonal antibodies, are described for the measurement of tissue plasminogen activator antigen (tPA:Ag) and tissue plasminogen activator-plasminogen activator inhibitor 1 (tPA-PAI-1) complexes.

WO 87/00549 relates to a method of producing monoclonal antibodies against endothelial type plasminogen activator inhibitor and immunologically similar inhibitors in which myeloma cells are fused with antibody-producing cells obtained from mammals which have been immunized with said plasminogen activator inhibitor or with antibody-producing cells, which in vitro have been immunized with said plasminogen activator inhibitor and in which the hybridomas producing antibodies against the above mentioned inhibitor are selected.

Accordingly, human tPA, human PAI.1 and PAI.1-tPA complex are important indices in the formation and dissolving of thrombus. If the concentrations of these factors in blood can be accurately measured, it would be possible to know the state of inhibiting thrombus formation and dissolving accurately.

3

It is an object of this invention to provide an immunological assay reagent system, a kit and a method for assaying PAI.1-tPA complex in a human assay sample.

Another object of this invention is to provide an immunological assay reagent system, a kit and a method for accurately assaying the amount of the PAI.1-tPA complex.

Another object of this invention is to provide an immunological assay reagent system, a kit and a method for accurately and easily assaying the PAI.1-tPA complex in a human assay sample with accuracy and stability.

Another object of the invention is to provide a monoclonal antibody to human PAI-1 used for assaying the PAI.1-tPA and a hybridoma capable of producing the monoclonal antibody.

Another object of this invention is to provide a monoclonal antibody to human tPA used for assaying the PAI.1-tPA complex, and a hybridoma capable of producing the monoclonal antibody.

Another object of this invention is to provide a method of diagnosing thrombosis by measuring the amount of the PAI.1-tPA complex in a human assay sample.

Another object of this invention is to provide a fibrinolysis promoter comprising a monoclonal antibody against human PAI.1.

Further objects of this invention will become apparent from the following detailed description.

The present invention relates to a monoclonal antibody JTI-3 against human plasminogen activator inhibitor 1 which was produced by hybridoma JTI-3 deposited as deposit No. BP-2317 at Fermentation Research Institute, Japan and to a monoclonal antibody JTI-4 against human plasminogen activator inhibitor 1 which was produced by hybridoma JTI-4 deposited as deposit No. BP-2318 at Fermentation Research Institute, Japan and to a monoclonal antibody JTA-1 against human plasminogen activator which was produced by hybridoma JTI-1 deposited as deposit No. BP-2316 at Fermentation Research Institute, Japan.

Moreover, the objects of this invention are achieved by a reagent system for immunologically assaying a PAI.1-tPA complex in a human assay sample by using a first antibody bound to an insoluble solid carrier and a labelled second antibody, one of the antibodies being monoclonal antibody JTI-3 or JTI-4 to human PAI.1 or an equivalent fragment thereof and the other being monoclonal antibody JTA-1 to human tPA or an equivalent fragment thereof.

Monoclonal antibodies to human PAI.1 so far reported include those obtained by using PAI derived from human plasma as an antigen [Urden, G. et al., Thromb. Haemost., 55, 383-387 (1986)], those obtained by using PAI derived from human placenta as an antigen [Phillips, M. et al., Thromb. Haemost.; 55, 213-217 (1986)], and those obtained by using PAI derived from human fibro-sarcoma cell line as an antigen [Nielsen, L. S. et al., Thromb. Haemost., 55, 206-212 (1986)].

The present inventors prepared many monoclonal antibodies using PAI derived from human vascular wall endothelial cells as an antigen, and also produced many monoclonal antibodies to human tPA.

The present inventors have extensively worked on the selection of means for assaying the PAI.1-tPA complex, screening of various monoclonal antibodies, and the combination of monoclonal antibodies, and found that not all of the many human PAI.1-MCA's and human tPA-MCA's so obtained can be utilized in the immunological assay of the PAI.1-tPA contemplated by this invention. In order to provide an assay sample which can be used in assaying the PAI.1-tPA complex with high assay accuracy and high stability, it is necessary to examine epitopes of human PAI.1-MCA and human tPA-MCA, their binding powers, affinity between human PAI.1-MCA and human tPA-MCA in their combination, and the compatibility between the above two monoclonal antibodies as the first antibody to be bound to an insoluble solid carrier or as the labelled second antibody in order to ensure that these characteristics should be within satisfactory ranges.

Investigations of the present inventors have led to the discovery of a combination of a monoclonal antibody JTI-3 or JTI-4 and a monoclonal antibody JTA-1 to tPA as monoclonal antibody which can be used to assay a tiny amount of the PAI.1-tPA complex present in a human assay sample with high sensitivity and stability.

There are the following two embodiments in the immunological assay of the PAI.1-tPA complex by the present invention.

(i) An embodiment in which the first antibody to be bound to the insoluble solid carrier is monoclonal antibody JTI-3 or JTI-4 against PAI.1, and the labelled second antibody is monoclonal antibody JTA-1 against tPA.

(ii) An embodiment in which the first antibody to be bound to the insoluble solid carrier is monoclonal anibody JTA-1 against tPA, and the labelled second antibody is monoclonal antibody JTI-3 or JTI-4.

The embodiment (i) is better in respect of sensitivity and stability, and the selection of the monoclonal antibody JTI-4 against PAI.1 gives favorable results. Thus, the most preferred embodiment in this invention is a combination of the monoclonal antibody against PAI.1 as the first antibody to be bound to the insoluble solid carrier and the monoclonal antibody against tPA as the labelled second antibody.

According to this invention, there are provided the monoclonal antibody JTI-3 and JTI-4 against PAI.1 and the monoclonal antibody against tPA for use in the immunological assay of the PAI.1-tPA complex, and hybridomas which produce these monoclonal antibodies. These monoclonal antibodies will be described below.

PAI.1-MCA.JTI-3

A hybridoma which produces monoclonal antibody JTI-3 against PAI.1 was deposited as FERM P-10405 on November 22, 1988 at Fermentation Research Institute, Japan which is an international depository under the Budapest Treaty. This deposit was then changed to an international deposit (International Deposit No. BP-2317) on March 2, 1989.

The subclass of PAI.1-MCA.JTI-3 is $IgG_1$. This monoclonal antibody has the property of recognizing an antigenic determining site at which when tPA binds to PAI.1, the binding of this monoclonal antibody against PAI.1 is not inhibited but when the monoclonal antibody first binds to PAI.1, tPA binding of tPA to PAI.1 is inhibited.

PAI.1-MCA-JTI-4

A hybridoma capable of producing the monoclonal antibody JTI-4 against PAI.1 was deposited at Fermentation Research Institute, Japan on November 22, 1988 under FERM P-10406. This deposit was changed to an international deposit (International Deposit No. BP-2318) on March 2, 1989.

The subclass of PAI.1-MCA-JTI-4 is $IgG_1$. Even when tPA binds to PAI-1, the binding of this monoclonal antibody to PAI.1 is not inhibited.

tPA-MCA-JTA-1

A hybridoma capable of producing the monoclonal antibody JTA-1 against tPA was deposited at Fermentation Research Institute, Japan on November 22, 1988 under FERM P-10399. This deposit was changed to an international deposite (International Deposit No. BP-2316) on March 2, 1989.

The binding of tPA-MCA-JTA-1 to tPA is not inhibited even when only PAI.1 binds to tPA.

In the immunological assay of the PAI.1-tPA complex in accordance with this invention, the above-mentioned monoclonal antibodies are used in combination. MCA fragments equivalent to these monoclonal antibodies may also be used. The "equivalent fragment of MCA" means a fragment which has the same binding characteristics as MCA, or a fragment-retaining the substantial binding ability of MCA. Specific examples of the fragment are a univalent antibody Fab, Fab', Facb and $(Fab')_2$. In particular, by using a Fab' fragment of MCA as the labelled antibody (second antibody), the PAI.1-tPA complex can be measured with very high sensitivity.

In the case of using tPA-MCA or PAI.1-MCA in the form of a Fab' fragment, the Fab' fragment can be prepared by methods known per se. For example, the Fab' fragment can be obtained by decomposing tPA-MCA or PAI.1-MCA with pepsin to form a $F(ab')_2$ fragment, and then subjecting it to a reducing treatment. For the method of preparing this Fab' fragment, reference may be made to the following publications.

(i) A. Nisonoff et al., Arch. Biochem. Biophys., 89, 230 (1960)

(ii) P. Parlam, J. Immunol., 131, 2893 (1983)

The human assay sample used in this invention for immunological assay of the PAI.1-tPA complex may generally be a clinical sample derived from human, for example, a body fluid which contain the PAI.1-tPA complex, such as blood in the form of serum or plasma, an articular fluid, a lymphatic fluid, a thymus fluid, ascites, an amniotic fluid, a cell tissue fluid, a bone marrow fluid or urine. Since the PAI.1-tPA complex is closely related to the formation or dissolving of thrombus, it is advantageous to use blood in the form of serum or plasma as the assay sample. In particular, a serum or plasma sample, particularly the latter, is suitably used for the diagnosis of thrombosis or the determination of cure of thrombosis.

The reagent system and kit and the assay method for the immunological determination of the PAI.1-tPA complex in this invention will be described below in detail.

(A-1) Reagent for immunological assay of PAI.1-tPA complex

In the reagent system of this invention for immunologically assaying the PAI.1-tPA complex in a human assay sample, it is preferable that the first antibody be PAI.1-MCA JTI-3 or JTI-4 or an equivalent fragment thereof, and the labelled second antibody be tPA-MCA JTA-1 or an equivalent fragment thereof. The use of JTI-4 as the PAI.1-MCA is especially preferred. In the most preferred embodiment, the first antibody is PAI.1-MCA JTI-4 (whole), and the second antibody is a Fab' fragment of tPA-MCA JTA-1.

The insoluble solid carrier used in the assay reagent system may be any of those insoluble solid carriers used generally in immunological assay reagents. The material from which the solid carrier is made may be a natural polymer or its derivative, or a synthetic polymer or its derivative, and may be organic or inorganic. Specifically, it is in the form of a plastic receptacle, a plastic bead, a latex bead, a glass bead, a plastic stick or a metal particle.

Examples of the polymers which constitute the solid carrier include natural polysaccharides or derivatives thereof, such as cellulose, Sephadex cellulose, carboxymethyl cellulose, nitrocellulose, cellulose acetate and dextran; inorganic polymers such as silica gel or glass; vinyl polymers such as polystyrene, polyethylene, polypropylene, ABS, polyvinyl fluoride, polyamines, methyl vinyl ether/maleic acid copolymer and ethylene/maleic acid copolymer; and condensation polymers, for example polyamides such as 6-nylon and 66-nylon, polyesters such as polyethylene terephthalate, and amino acid polymers.

Examples of more specific forms of the insoluble solid carrier are a test tube, a microtiter plate, a bead, a stick and a membrane.

Investigations of the present inventors have shown that if an insoluble solid carrier having a smooth specular surface is used in the assaying of the PAI.1-tPA complex, the non-specific adsorption of the labelled antibody to the solid carrier is inhibited; consequently, the sensitivity of assaying the PAI.1-tPA complex is increased and the assay is stabilized.

The insoluble solid carrier having a specular surface advantageously has a centerline average roughness (Ra) at its surface of not more than 1.5 micrometers. Polystyrene beads may be used preferably as the insoluble solid carrier having a specular surface.

The centerline average roughness (Ra) is determined as follows:- A portion having a measured length L is extracted from the roughness curve in the direction of its center line. The center line of the extracted portion is made X axis, and the machine direction is made Y axis. When the roughness curve is expressed as $y = f(x)$, the centerline rouhgness (Ra) is given by the following equation (in $\mu$m (microns)). It is described in JIS B 0601-1982. JIS B 0601-1982 corresponds to ANSI B 46.1-1978 (USA) and R 468-1966 (ISO).

The first antibody may be immobilizd to the insoluble solid carrier by methods known per se, for example, a physical adsorption method (such as by immersing the insoluble solid carrier in a solution of the first antibody); an ionic bonding method (such as by using an ion exchange resin, or a solid carrier having an ionizable functional group such as an amino, carboxylic acid, sulfonic acid or phosphoric acid group); a covalent bonding method by a chemical reaction, such as a carboxyl chloride method, a carbodiimide method, a maleic anhydride derivative method, an isocyanate derivative method, a cyanogen bromide-activated polysaccharide method, a diazo method, an active ester method, or a carrier binding method by crosslinking reagents (the crosslinking agents include, for example, glutaraldehyde, hexamethylene isocyanate or succinimide-maleimide compound), or a method of binding via a substance having no ability to bind to tPA or PAI.1 but being capable of binding to the first antibody by a biological reaction, for example, a method involving using a protein A-bound solid carrier.

The labelling substance to be bound to the second antibody in this invention may be, for example, an enzyme, a fluorescent substance, a luminescent substance or a radioactive substance which are usually employed in immunological assay. Specific examples include enzymes such as lysozyme maleate dehydrogenase, glucose-6-phosphate dehydrogenase, peroxidase, glucose oxidase, alkaline phosphatase, luciferase and beta-galactosidase; fluorescent substances such as fluorescein, rhodamine, umbelliferone, lanthanide and chelate; luminescent substances such as luminol, acridinium ester and luciferin; and radioactive substances such as iodine-125, iodine-131, tritium and carbon-13. The enzymes are preferred among them as the labelling substance. The bonding of the labelling substance to the second antibody or its fragment may be carried out by ordinary methods, for example, by a glutaraldehyde method, a periodic acid method or a maleimide method, the maleimide method being prefered.

Maleimidization of the enzyme may be carried out by a known method (for example, "Enzyme Immunoassay", 3rd edition, pages 80-83, 1989, edited by Eiji Ishikawa, published by Igaku Shoin). It can be carried out by using, for example, succinimidyl 4-(N-maleimidomethyl)-cyclohexane carbonate (SMCC), sulfosuccinimidyl 4-(N-maleimidomethyl)cyclohexane carbonate (sulfo SMCC), succinimidyl metamaleimide benzoate (MBS) and succinimidyl 6-maleimidehexanoate (EMCS).

The reaction between the second antibody or its fragment and the maleimidized labelling substance is carried out generally by the method described in the above-cited literature reference, for example at a reaction temperature of 4 to 30 °C, for a reaction period of 20 hours using the second antibody or its fragment and the labelling substance in a mole ratio of 1:1. This reaction mainly gives the antibody or its fragment labelled with one molecule, per molecule of the antibody or its fragment, of the labelling substance through the sulfur atom of the antibody or its fragment.

In the present invention, the second antibody labelled with at least 1.5 molecules on an average, per molecule of a Fab' fragment of the second antibody through the sulfur atom of the Fab' fragment of the second antibody is preferably used as the labelled antibody because it leads to high sensitivity assay in accordance with this invention. If the amount of the labelling substance bound to the antibody is less than 1.5 molecules, the labelled antibody has low activity and is unsuitable for high sensitivity assay. Preferably, the amount of the labelling substance bound to the antibody is at least 1.8 molecules per molecule of the Fab' fragment. There is no particular upper limit, but it is preferably up to 5. Such a multilabelled antibody is obtained by isolation and purification from the reaction mixture obtained by the above reaction. To obtain the multilabelled antibody in good yields, the reaction is preferably carried out at a reaction temperature of at least 25 °C for a period of at least 24 hours while adjusting the molar ratio of the Fab' fragment to the maleimidized labelling substance to 1:at least 4. The buffer used in the reaction may be any buffer which does not inhibit the reaction. Usually, 0.01-0.5 M phosphate buffer having a pH of 5.5 to 8.0 is preferably used. For stabilization of the Fab' fragment, it may contain about 10 millimoles of EDTA, and this gives better results. The multilabelled antibody can be isolated and purified by gel chromatography. Ultrogel™ AcA44 (LKB) may be used as a carrier for the gel chromatography. However, because of better separation, HPLC on a TSK Gel-3000SW (a product of Toso) column or a DIOL-200 (a product of Yamamura Chemical Laboratory) column is preferred.

The number of molecules of the labelling substance can be determined from the measurement of the molecular weight, absorbance, fluorescence intensity, and enzyme activity of the labelling substance. For example, if the labelling substance is peroxidase, the number of molecules of the labelling substance can be determined by measuring the absorbance at 280 nm attributed to peroxidase and the Fab' fragment and the absorbance at 403 nm attributed to peroxidase.

The above method can give a labelled Fab' fragment which can assay the PAI.1-tPA complex with high sensitivity.

(A-2) Kit for assaying PAI.1-tPA complex

The present invention provides an immunological assay kit for assaying the PAI.1-tPA complex in a human assay sample, comprising a combination of
(i) a first antibody bound to an insoluble solid carrier,
(ii) a labelled second antibody,
(iii) a dissolving agent,
(iv) a washing agent, and
(v) where an antibody labelled with an enzyme is used, a substrate for measuring the enzyme activity and a reaction stopper,
one of the antibodies being monoclonal antibody JTI-3 or JTI-4 against PAI.1 or an equivalent fragment thereof and the other being monoclonal antibody JTA-1 against tPA or an equivalent fragment thereof.

The first antibody (i) bound to an insoluble solid carrier and the labelled second antibody (ii) may be the same as these described with regard to (A-1) reagent system for assaying PAI.1-tPA complex.

The dissolving agent (iii) constituting the assay kit of this invention may be any of those dissolving agents whicn are normally used in immunological assay. The dissolving agent used in this invention may be one which does not adversely affect the immunological reaction, and for example, a phosphate buffer, a Tris-HCl buffer, and an acetate buffer which have a pH in the range of 6.0 to 8.0 are mainly used.

The washing agent (iv) may be any of those which are normally used in immunological assay. Examples include physiological saline, a phosphate buffer, a Tris-HCl buffer, and mixtures of these. Mixtures of these with nonionic detergent such as Triton™ X-100, Tween™ 20 or Brij™ 35 or ionic detergent such as sodium dodecylsulfate may also be used. The amount of these detergents is desirably 0.5 to 0.001 %, preferably 0.1 to 0.01 %.

Where an enzyme is used as the labelling substance for the second antibody in the above assay kit, the substrate (v) for measuring the enzyme activity and a reaction stopper are used in combination with the above materials. Known substrates and reaction stoppers may be used according to the type of the enzyme as the labelling substance. For example, as a substrate for peroxidase, 2,2'-azino-di-[3-ethylbenthiazolinsulfonic acid (ABTS), o-phenylenediamine (OPD), 3,3',5,5'-tetramethylbenzidine (TMB) are used. As a reaction stopper for peroxidase, $H_2SO_4$, HCl, $CH_2COOH$ or glycine buffer (pH 10.3) are used.

As a substrate for alkaline phosphatase, 4-nitrophosphate or NADP are used. As a stopper for alkaline phosphatase, 1N NaOH is used.

As a substrate for $\beta$-galactosidase, 2-nitrophenyl-$\beta$-galactosidase, etc. is used. As a stopper for $\beta$-galactosidase, 0.1 M $Na_2CO_3$ is used.

In the kit of this invention, a non-specific adsorption inhibitor may be incorporated in it in order to increase the sensitivity of the PAI.1-tPA complex and permit determination of a tiny amount of it in the sample. The inhibitor may be an agent which prevents or suppresses the non-specific adsorption of the antigen or antibody in the sample to the assay receptacle or the insoluble solid carrier. Hence, the incorporation of the non-specific adsorption inhibitor in the kit of this invention is favorable for increasing the assay sensitivity and accuracy of the PAI.1-tPA complex. The non-specific adsorption inhibitor exhibits its action when it is present in the immunological reaction solution in the assay of the PAI.1-tPA complex. Accordingly, it is combined with the kit in various modes. For example, the non-specific adsorption inhibitor may be used in the form of a solution as one element of the kit. Alternatively, it may be present together with the first antibody (i) bound to an insoluble solid carrier, the labelled second antibody (ii) or the dissolving agent (iii). It may also be combined as one elemnt of the kit such way that it can be present in the assay sample.

(A-3) Method for immunologically assaying a PAI.1-tPA complex in a human sample

According to this invention, there is also provided a method for immunologically assaying a PAI.1-tPA complex in a human sample, which comprises using a monoclonal antibody JTI-3 or JTI-4 against PAI.1 or an equivalent fragment thereof, or a monoclonal antibody JTA-1 against tPA or an equivalent fragment thereof as either a first antibody or a second antibody, and

(i) bringing the human assay sample into contact with the first antibody bound to an insoluble solid carrier, then washing the carrier and bringing it into contact with a labelled second antibody, or

(ii) bringing the human assay sample into contact with the first antibody bound to an insoluble solid carrier and the labelled second antibody in the same assay system.

This assay method is a technique generally known as the sandwich method except that the above antibodies or fragments thereof selected in this invention are used. Accordingly, the PAI.1-tPA in the human assay sample can be measured by the known technique by using the first antibody bound to the insoluble solid carrier, the labelled second antibody, the dissolving agent and the washing agent which are described in the above sections (A-1) and (A-2).

The assay can be performed by a two-step method, as described in (i), which comprises reacting the sample with the first antibody bound to the insoluble solid carrier, washing the sample, then reacting it with the labelled second antibody, and washing the sample to permit coloration reaction; or by a one-step method, as described in (ii), which comprises allowing the sample, the first antibody bound to the insoluble solid carrier and the labelled second antibody to be present together in a reaction solution, and incubating the reaction solution for a predetermined period of time, washing the reaction mixture to permit coloration reaction.

According to this invention both of the one-step method and the two-step method can determine the PAI.1-tPA complex in the human assay sample with good sensitivity. When the human assay sample contains a relatively large amount of tPA or the presence of a large amount of tPA in the sample is anticipated, the two-step method is generally preferred.

Both of the one-step method and the two-step method are carried out at a temperature of generally not more than 50 °C, preferably about 4 °C to about 45 °C, for a period of generally about 5 minutes to about 20 hours, preferably about 10 minutes to about 3 hours.

As stated above, the presence of the non-specific adsorption inhibitor in the reaction system can increase the sensitivity and accuracy of assaying the PAI.1-tPA complex. Investigations of the present inventors have shown that the use of a predetermined proportion of a certain kind of protein described below as the non-specific adsorption inhibitor in the assay of the PAI.1-tPA complex produces desirable results.

Specifically, when the assay method of this invention is carried out by causing a protein having a molecular weight of 16,000 to 50,000, preferably 20,000 to 46,000 and an isoelectric point of 1.0 to 5.0, preferably 1.2 to 4.8, to be present in the immunological reaction solution, and adjusting the final concentration of the protein in immunological reaction solution to 0.02 to 0.9 % by weight, the non-specific adsorption can be inhibited. Thus, the background is very low, and high sensitivity is easy to obtain. Examples of the protein having the above properties are casein, beta-casein, alpha-casein, pepsin, ovoglycoprotein and orosomucoid. Mixtures containing these proteins may also be used. For example, there may be used a non-specific adsorption inhibitor composed of 10 to 60 % by weight, preferably 20 to 50 % by weight, of the above protein, 30 to 80 % by weight, preferably 40 to 60 % by weight, of a sugar such as

8

EP 0 339 302 B1

lactose as main components and fats (for example 0.5 to 2 % by weight), ashes (for example, 5 to 12 % by weight), and water (for example, 2 to 8 % by weight). Skimmed milk is a typical example of this mixture. Skimmed milk contains casein as a prortein, and has better dispersibility in the immunological reaction solution than casein alone. Skimmed milk has a high inhibition effect of non-specific binding (NSB) per unit weight of the protein, and has good storage stability at 4 °C (difficulty of avoiding precipitation). The skimmed milk used in this invention may be any defatted milk from any source. High sensitivity is obtained by adding skimmed milk to the immunological reaction solution and adjusting its final concentration in the solution to 0.02 to 0.8 % by weight. If its final concentration is lower than 0.02 % by weight, no sufficient inhibitory effect can be obtained. On the other hand, if the concentration of the inhibitor is higher than 0.8 % for skimmed milk, and 0.9 % by weight for the other proteins mentioned above, the specific reaction is also inhibited undesirably.

(B) Immunological adsorbent

An immunological adsorbent obtained by binding monoclonal antibody JTI-3 or JTI-4 against PAI.1 or a fragment thereof to an insoluble solid carrier is novel. It is used not only as an element of the assay reagent system (A-1) but also for separating and recovering the individual components from (a) a mixture containing PAI.1 and the PAI.1-tPA complex, (b) a mixture containing tPA and the PAI.1-tPA complex or (c) a mixture containing PAI.1, tPA and the PAI.1-tPA complex. The method of separating and recovering the individual components using the immunological adsorbent will be described.

The mixtures (a) to (c) may be the human assay sample described above, particularly blood such as serum or plasma, or a mixture which is obtained by adding human tPA to the culture product (such as the culture supernatant) of human PAI.1-producing cells such as human vascular wall endothelial cells. So long as these mixtures contain PAI, tPA and/or the PAI.1-tPA complex, they may be used irrespective of the contents of these factors.

Separating and recovery method I

This method comprises
(1) contacting a starting mixture containing PAI.1 and a PAI.1-tPA complex with the immunological adsorbent to bind PAI.1 and the PAI.1-tPA adsorbent selectively,
(2) separating the adsorbent from the mixture,
(3) contacting the separated adsorbent with an eluent to elute PAI.1 and the PAI.1-tPA complex in the eluent,
(4) thus obtaining a mixed solution containing PAI.1 and the PAI.1-tPA complex,
(5) contacting the resulting mixed solution with an immunological adsorbent obtained by binding an antibody to tPA or a fragment thereof to an insoluble solid carrier to bind the PAI.1-tPA complex selectively to the adsorbent,
(6) separating the adsorbent having PAI.1-tPA complex bound thereto from the mixed solution,
(7) contacting the separated adsorbent with an eluent to elute the PAI.1-tPA complex in the eluent,
(8) recovering the PAI.1-tPA complex from the eluent, and
(9) if required, recovering PAI.1 from the solution separated in step (6).
The "antibody against tPA" in step (5) in the above method I is monoclonal antibody JTA-1 described above.

PAI.1 may be recovered from the mixed solution separated in step (6), and this method is also encompassed within the present invention.

A solution of sodium thiocyanate or urea, for example, may be used as the eluent used in steps (3) and (7). 0.2 M glycine HCl (PH 2.5), 0.2M acetic acid, 1 M $Na_4OH$, 3 M $NH_4SCN$ and 6 M urea are used as the eluent.

The method I makes it possible to separate the PAI.1-tPA complex and/or PAI.1 of high purity at a high recovery ratio from the starting mixture containing PAI.1 and the PAI.1-tPA complex (which may further contain tPA). The PAI.1-tPA complex and/or PAI.1 so recovered can be used effectively as a standard sample for immunological assay or for other experiments.

9

Separating and recovery method II

This method comprises

(1) contacting a starting mixture containing tPA and a PAI.1-tPA complex with an immunological adsorbent obtained by binding an antibody to tPA or an equivalent fragment thereof to an insoluble solid carrier thereby bind tPA and the PAI.1-tPA complex selectively to the immunological adsorbent,

(2) separating the adsorbent from the mixture,

(3) contacting the separated adsorbent with an eluent to elute tPA and the PAI.1-tPA complex in the eluent,

(4) thus obtaining a mixed solution containing tPA and the PAI.1-tPA complex,

(5) contacting the resulting mixed solution with an immunological adsorbent obtained by binding monoclonal antibody JTI-3 or JTI-4 against to PAI.1 or a fragment thereof to an insoluble solid carrier to bind the PAI.1-tPA complex selectively to the adsorbent,

(6) separating the adsorbent having the PAI.1-tPA complex bound thereto from the mixed solution,

(7) contacting the separated adsorbent with an eluent to elute the PAI.1-tPA complex in the eluent,

(8) recovering the PAI.1-tPA complex from the eluate, and

(9) if required, recovering tPA from the solution separated in step (6).

The "antibody against tPA" in step (1) in the above method I is monoclonal antibody JTA-1 described above.

tPA may be recovered from the mixed solution separated in step (6), and this method is also encompassed within the present invention.

The eluents used in steps (3) and (4) may be the same as used in the method I described above.

The method II makes it possible to separate the PAI.1-tPA complex and/or tPA of high purity at a high recovery ratio from the starting mixture containing PAI.1 and the PAI.1-tPA complex (which may further contain tPA). The PAI.1-tPA complex and/or PAI.1 so recovered can be used effectively as a standard sample for immunological assay or for other experiments.

(C) Diagnosis of the condition of thrombosis

By measuring the amount of the PAI.1-tPA complex in a human assay sample, particularly a human blood sample, by the method described in (A-3) above, the condition of thrombus of the sample donor can be diagnosed. When the PAI.1-tPA complex is contained in concentrations above a certain limit, it can be determined that the donor is suffering from thrombosis or is in the course of progressing to thrombosis. When the amount of the above complex in a blood sample of a person who is suffering from thrombosis and treated is measured and found to be below a certain limit (about 9 ng/ml), it can be determined that this thrombosis is cured.

Furthermore, by measuring the amount of the PAI.1-tPA complex in a human assay sample, especially a human blood sample, in accordance with the method described under section (A-3) and the total amount of PAI.1 and the PAI.1-tPA complex in the sample in accordance with the method described under section (B), the condition of thrombosis can be more accurately diagnosed from the two measured amounts or from the total amount of PAI.1 calculated.

The method of preparing PAI.1-MCA in this invention will be described in detail.

A. Isolation and purification of an antigen

PAI used as an antigen is isolated and purified from the culture supernatant of human vascular wall endothelial cells by applying the method of van Mourik, J. A. et al. [see van Mourik J. A. et al., J. Biol. Chem., 259, 14914-14921 (1984)].

B. Immunization of mice with PAI.1

Female Balb/C mice can be used, but mice of another strain may also be used. The immunization plan and the concentration of PAI.1 should be selected so that a sufficient amount of antigenically stimulated lymphocytes may be formed. For example, the mice are immunized three times intraperitoneally with 50 micrograms of PAI.1 at intervals of 21 weeks. Several days after the final immunization, the spleen cells are taken out for fusion.

10

C. Cell fusion

The spleen of the immunized mice is aseptically taken out, and a single cell suspension was prepared from it. The spleen cells were fused with mouse myeloma cells from a suitable cell line in the presence of a suitable fusion promoter. The preferred ratio of the spleen cells to the myeloma cells is in the range of from about 20:1 to about 2:1. The use of 0.5 to 1.5 ml of a fusion medium per about $10^8$ spleen cells is suitable.

The mouse myeloma cells used for cell fusion are well known. In the present invention, P3-X63-Ag8-U1 cells (P3-UI1) [Yelton, D. T. et al., Current Topics in Microbiology and Immunology, 81, 1 (1978)] are used.

As a preferable fusion promoter for the cell fusion, polyethylene glycol having an average molecular weight of 1000 to 4000 may be advantageously used. Other fusion promoters known in the art may also be used. In the present invention, polyethylene glycol having an average molecular weight of 4000 is used.

D. Selection of fused cells

In a separate receptacle (such as a microtiter plate), a mixture of the unfused spleen cells, the unfused mouse myeloma cells and the fused hybridoma cells is diluted with a selective medium which does not support the unfused mouse myeloma cells, and cultured for a period sufficient to cause the unfused cells to die away (about 1 week). The medium is drug resistant (for example, 8-azaguanine resistant) and does not support the unfused mouse myeloma cells (for example, HAT medium). In this selective medium, the unfused myeloma cells die. The unfused spleen cells are non-tumoral cells, and therefore die after a predetermined period of time (about 1 week later). The fused cells can survive in the selective medium since they have both the tumoral nature of the parent myeloma cells and the characteristics of the parent spleen cells.

E. Determination of an antibody against PAI.1 in a receptacle

After the hybridoma cells are detected, the culture supernatant is collected and screened for an antibody against PAI.1 by enzyume-linked immunosorbent assay.

F. Cloning of hybridoma cells capable of producing an antibody having activity on PAI.1

When the hybridoma cells capable of producing the desired antibody by a suitable method (for example, a limiting dilution method), the antibody formed by any of two different ways. Firstly, by cultivating the hybridoma cells in a suitable medium for a predetermined period of time, the monoclonal antibody produced by the hybridoma cells can be obtained from the culture supernatant. Secondly, the hybridoma cells can be injected intraperitoneally into a syngenic or semi-syngenic mouse. After a predetermined period of time, the monoclonal antibody produced by the hybridoma cells can be isolated from the blood and ascites of the host animal.

G. Separation of PAI.1 from a PAI.1-containing mixture

The MCA against PAI.1 is fixed or bounds to an isoluble carrier to obtain an adsorbent. The insoluble carrier used at this time may be any of those which are generally used as a substrate of an assay reagent or a kit containing MCA. For example, it may be formed of Sepharose, polyacrylamide, cellulose, dextran or a maleic acid polymer or a mixture thereof, and may be in the form of a powder, granules, pellets, beads, film or a fiber. Advantageously, a plate having a number of depressions (wells) for use in assaying or separation of plasma or its fractionated components may be used as the insoluble solid carrier.

When the PAI.1-containing mixture is brought into contact with the adsorbent, MCA fixed to the adsorbent binds to PAI.1, and consequently, PAI.1 binds to the adsorbent. PAI.1 can be separated and removed from the mixture.

If possible, the remaining mixture is removed by washing. PAI.1 bound to the adsorbent is then detached from the adsorbent under acidic conditions (0.2 M glycine HCl).

Thus, by using the adsorbent having MCA bound thereto, the removal of PAI.1 from a mixture containing it, the separation and purification of PAI.1 from the mixture, and the measurement of the content of PAI.1 in the mixture can be achieved by a very simple operation.

H. Separation of human PAI.1-tPA complex from a mixture containing the PAI.1-tPA complex

MCA against PAI.1 is fixed or bound to an insoluble carrier which may be the same as described in section G above.

When the mixture containing the PAI.1-tPA complex is brought into contact with the resulting adsorbent, MCA fixed to the adsorbent binds to the PAI.1-tPA complex and consequently, the PAI.1-tPA complex binds to the adsorbent. This enables separation and removal of the PAI.1-tPA complex. As above, the PAI.1-tPA complex is bound to the adsorbent and if possible, the remaining mixture is removed by washing. The PAI.1-tPA complex bound to the adsorbent is removed under acidic conditions (0.2 M glycine HCl) and can thus be isolated.

By using the adsorbent having MCA adsorbed on it, the removal of the PAI.1-tPA from the mixture containing it, the separation and purification of the PAI.1-tPA complex from the mixture and the measurement of the content of the PAI.1-tPA complex in the mixture can be achieved by a very simple operation.

BRIEF DESCRIPTION OF THE DRAWING

Figure 1 shows the results of immunoblotting which represent the bindability of MCA-JTI-2 against PAI.1 and the PAI.1-tPA complex in Example 1-(2);
Figure 2 shows the bindability of each of the antibodies in Example 3 to the PAI.1-tPA complex;
Figure 3 shows the binding ability of the combination of antibodies in Example 4;
Figure 4 shows the effects of free tPA in Example 5 on the assay system;
Figure 5 shows the effects of free PAI.1 on the assay system in Example 6;
Figure 6 shows the effect of free PAI.1 on the assay system when beads are used as a solid carrier in Example 7;
Figure 7 shows the results of measuring the total amount of PAI.1 antigen in Example 8;
Figure 8 shows the ability of anti-PAI.1 MCA to inhibit the formation of the PAI.1-tPA complex in Example 11;
Figure 9 shows the fibrinolysis promoting effect of MCA.JTI-3 in Example 12;
Figure 10 shows the results of measuring the concentration of the PAI.1-tPA complex in plasma samples of a normal subject and various patients in Example 13;
Figure 11 shows the results of purifying PAI.1 in Example 14;
Figure 12 shows the results of purifying the PAI.1-tPA complex in Example 15;
Figure 13 shows a calibration curve in the assay system in Example 16;
Figure 14 shows a calibration curve in the assay system of Example 18;
Figure 15 shows the relation between the surface roughness of beads and non-specific adsorption in Example 19;
Figure 16 shows the reaction yield of the labelled antibody in Example 20 with the lapse of time;
Figure 17 shows the calibration curves prepared in Example 21; and
Figure 18 shows the calibration curves prepared in Example 22.

The following Examples illustrate the present invention in more detail. Throughout, the examples of the antibodies other than JTJ-3, JTJ-4 and JTJ-1 are mentioned for comparative purpose.

EXAMPLE 1-(1)

Isolation and purification of an antigen

PAI.1 used as an antigen was isolated and purified from the culture supernatant of human vascular wall endothelial cells by applying the method of van Mourik J. A. [see van Mourik J. A. et al., J. Biol. Chem., 259, 14914-14921 (1984)].

Specifically, the culture supernatant of human vascular wall endothelial cells free from bovine serum was brought into contact with a Con A Sepharose adsorbent to permit adsorption of a fraction containing PAI.1 in the supernatant. The adsorbent was washed, and then eluted with a buffer containing 1 M alpha-methylmannoside. The eluted fraction was subjected to molecular weight fractionation by an SDS-polyacrylamide slab gel electrophoresis. A gel in a molecular weight portion corresponding to PAI.1 was cut out, and from this gel slice, the protein was extracted to form an antigen.

EXAMPLE 1-(2)

Obtaining MCA

Two female Balb/C mice (6 weeks) were immunized four times at an interval of 14 days with PAI.1 purified by the method of Example 1-(1). The first immunization was effected by intraperitoneally administering an emulsion obtained by mixing 50 micrograms of PAI.1 dissolved in PBS with an equal amount of complete Freund's adjuvant so that the amount of the emulsion administered per mouse was 50 micrograms. In the second and third immunizations, a mixture of 50 micrograms of PAI.1 with Freund's incomplete adjuvants was intraperitoneally administered. In the final immunization, a PBS solution of 10 micrograms of PAI.1 was administered from the tail vein of the mice. Three days after the final immunization, the spleen cells of the immunized mice was taken out for cell fusion.

The spleen cells of the immunized mice were mixed with myeloma cells (P3U1) of mice of the same strain at a ratio of from about 2:1 to about 15:1, and fused in the presence of 50 % polyethylene glycol 4000 (Merck) as a fusion promoter by the method of Koehler and Milstein [Koehler and Milstein, Nature, 256, 495-497 (1975)]. The fused cells were suspended in a 10 % FCS-RPMI-1640 medium to a cell concentration of $1 \times 10^6$ cells/ml. The suspension was added to a 96-well microtiter plate (Coster) at a rate of 100 microliters per well.

The fused cells were incubated in a $CO_2$ incubator (5 % $CO_2$, 37 °C), and the medium was replaced by a medium containing hypoxanthine, aminopterin and thymidine (HAT medium). The fused cells were grown in the HAT medium. Hybridoma cells composed of the spleen cells and the myeloma cells were screened.

Antibody in the culture supernatant of the hybridomas was detected by ELISA using microtiter plates coated with the antigen PAI.1. As a second antibody, rabbit anti-mouse IgG antibody labelled with horseradish peroxidase (abbreviatd HRP) was used.

The formation of colonies was observed in 511 wells out of 570 wells to which the fused cells were added. Of these wells, only 8 wells showed the production of antibody, as shown in Table 1. Cloning by a limiting dilution method was carried out twice in these antibody-producing wells, and five clones were obtained. Monoclonal antibodies produced from these clones were named JTI-1, JTI-2, JTI-3, JTI-4 and JTI-5. The clones obtained were suspended in 90 % FCS-10 % DMSO and stored in liquid nitrogen. The monoclonal antibodies produced by these clones were grown in the abdominal cavities of Balb/C mice and purified from their ascites using a protein A-Sepharose 4B column.

Table 1

| | |
|---|---|
| Spleen cells (cells/ml) | $9.0 \times 10^7$ |
| Myeloma cells (cells/ml) | $1.3 \times 10^7$ |
| Ratio of the spleen cells to the myeloma cells | 6.9 |
| Number of cells per well | $1.58 \times 10^5$ |
| Number of wells | 570 |
| Number of positive wells which formed colonies | 511 (89.6 %) |
| Number of positive wells which produced antibodies | 8 (1.57 %) |

Bindability of MCA to PAI.1 activated with sodium dodecylsulfate by an enzyme immunoassay method

The bindability of MCA to PAI.1 activated with sodium dodecylsulfate (SDS for short) was determined by using an antigen-fixed enzyme immunoassay method. SDS was added to a final concentration of 0.1 % to a quiescent PAI.1 solution, and the mixture was incubated at 37 °C for 1 hour. Then, Triton X-100 was added to a final concentration of 1 %. The mixture was left to stand for 1 hour on ice. This operation gave PAI.1 activated with SDS.

Enzyme immunoassay was carried out using SDS-activated PAI.1 as an antigen and for comparison, the PAI.1-tPA complex and quiescent PAI.1 as antigens.

Polystyrene plastic wells were coated overnight at 4 °C with each of the antigens in a protein concentration of 1 microgram/ml, and the binding ability of MCA to each of the antigens was examined. The wells were washed, blocked with BSA, and incubated at 37 °C for 2 hours with 5 monoclonal antibodies prepared to a protein concentration of 5 micrograms/ml. After washing, the wells were incubated together with HRP (horseradish peroxide)-labelled rabbit anti-mouse antibody. After washing, the peroxidase activity remaining in the wells was determined, and the bindability was examined. Five monoclonal antibodies showed bindability to the PAI.1-tPA complex, quiescent PAI.1 and SDS-activated PAI.1. JTI-1 showed stronger bindability to quiescent PAI.1, and JTI-2 showed stronger bindability to SDS-activated PAI.1 JTI-3, JTI-4 both bound strongly to the three antigens. JTI-5 bound to the PAI.1-tPA complex.

Bindability of MCA to SDS-activated PAI.1 by immunoblotting

300 ng of each of PAI.1 and the PAI.1-tPA complex was subjected to SDS slab gel electrophoresis, and thereafter, the protein was transferred electrically to a nitrocellulose membrane. The nitrocellulose membrane was blocked with BSA, washed, and maintained at room temperature for 2 hours in a solution of each of JTI-1 to JTI-5 prepared so as to provide a final concentration of 10 micrograms/ml. After washing, the solution was incubated with HRP-labelled rabbit antimouse antibody. After washing, the activity of peroxidase in the HRP-labelled antibody remaining bound to the antigen was detected. The results are shown in Table 2. JTI-2 to JTI-4 showed bindability both to PAI.1 alone and the PAI.1-tPA complex.

Table 2

| Clone | Subclass | | Binding by EIA | | | Binding by immunoblotting | |
|---|---|---|---|---|---|---|---|
| | H-chain | L-chain | qPAI | aPAI | Complex | aPAI | Complex |
| JTI-1 (FERM P-10403) | IgG1 | k | ++ | + | + | - | - |
| JTI-2 (FERM P-10404) | IgG1 | k | + | ++ | ++ | + | + |
| JTI-3 (FERM P-10405) | IgG1 | k | ++ | ++ | ++ | + | + |
| JTI-4 (FERM P-10406) | IgG1 | k | ++ | ++ | ++ | + | + |
| JTI-5 (FERM P-10407) | IgG2a | k | ± | ± | + | - | - |

++: bound very strongly

+: bound

±: bound weakly

-: did not bind

qPAI: quiescent PAI.1

aPAI: SDS-activated PAI.1

Complex: PAI.1-tPA complex

When the bindability of MCA to the antigen was examined by an antigen fixing method, the dissociation constants (Kd for short) shown in Table 3 were obtained.

Table 3

|  | qPAI | Complex |
|---|---|---|
| JTI-1 | $9.1 \times 10^{-9}$ M | $4.4 \times 10^{-9}$ M |
| JTI-2 | $4.7 \times 10^{-9}$ M | $1.03 \times 10^{-8}$ M |
| JTI-3 | $2.1 \times 10^{-9}$ M | $5.0 \times 10^{-8}$ M |
| JTI-4 | $9.2 \times 10^{-9}$ M | $2.8 \times 10^{-8}$ M |
| JTI-5 | $1 \times 10^{-5}$ M < | $1 \times 10^{-5}$ M < |

Bindability of JTI-2 by immunoblotting

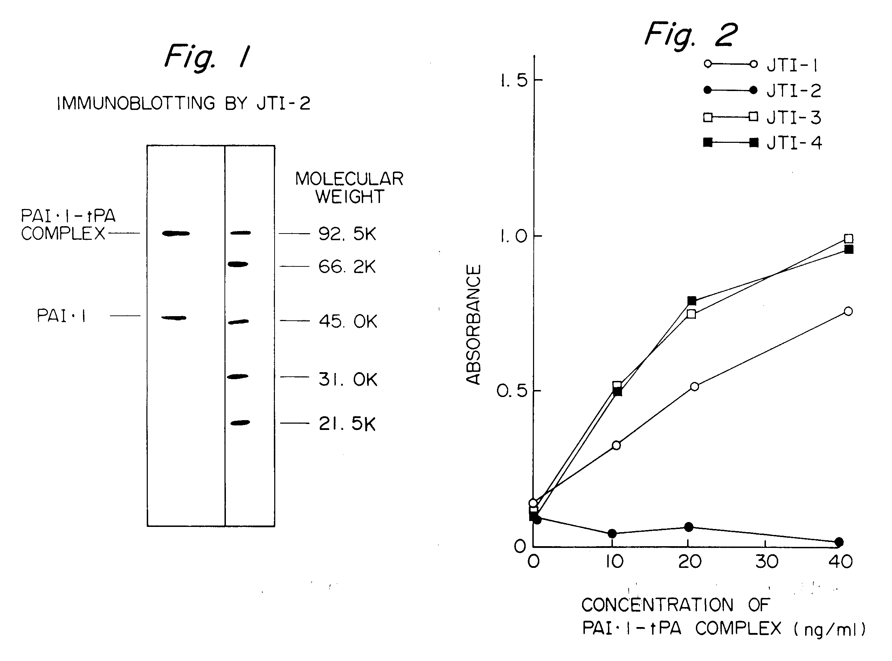

300 ng of PAI.1 or the PAI.1-tPA complex was subjected to SDS slab gel electrophoresis, and then the protein was electrically transferred to a nitrocellulose membrane. The nitrocellulose membrane was blocked with BSA, washed, and maintained at room temperature for 2 hours in a solution of JTI-2 prepared so as to provide a protein concentration of 10 micrograms/ml. After washing, the solution was incubated with HRP-labelled rabbit antimouse antibody. After washing, the peroxidase activity of HRP-labelled antibody remaining bound to the antigen on the nitrocellulose was detected. The results are shown in Figure 1. JTI-2 showed bindability both to PAI.1 alone and the PAI.1-tPA complex.

EXAMPLE 2

Preparation of tPA-MCA

tPA isolated and purified from the culture supernatant of human melanoma cells by applying the method of Rijken D. C. et al. [Rijken D. C. et al., J. Biol. Chem., 258, 7035-7041 (1981)] was used as an antigen, and in the same way as in Example 1, the following tPA-MCA-producing hybridomas and tPA-MCA were prepared.

The resulting monoclonal antibodies were named JTA-1, JTA-2, JTA-3 and JTA-4, respectively. The bindability of the monoclonal antibodies to tPA or PAI.1-tPA complex was examined by enzyme immunoassay and immunoblotting in the same way as in Example 1. The results are shown in Tables 4 and 5.

EP 0 339 302 B1

Table 4

| Clone | Subclass | | Binding by EIA | | | Binding by immunoblotting | | | |
| | | | | | | Non-reduced condition | | Reduced condition | |
| | H-chain | L-chain | tPA | H-chain of tPA | PAI.1-tPA | H-chain of tPA | Complex | H-chain of tPA | Complex |
|---|---|---|---|---|---|---|---|---|---|
| JTA-1 (FERM P-10399) | IgG1 | k | + | + | + | + | + | − | − |
| JTA-2 (FERM P-10400) | IgG1 | k | + | + | + | + | + | − | − |
| JTA-3 (FERM P-10401) | IgG1 | k | + | − | + | − | + | − | + |
| JTA-4 (FERM P-10402) | IgG1 | k | + | + | + | + | + | − | − |

+: bound

−: did not bind

Table 5

| Kd (dissociation constant) x $10^{-9}$ M | | | |
|---|---|---|---|
| Monoclonal antibody | Single-stranded tPA | Double-stranded tPA | Complex |
| JTA-1 | 7.4 | 3.4 | 36 |
| JTA-2 | 3.7 | 1.5 | 24 |
| JTA-3 | 9.8 | 6.7 | 6.7 |
| JTA-4 | 5.4 | 9.2 | 0.7 |
| Complex: PAI.1-tPA complex | | | |

EXAMPLE 3

Examination of various antibody combinations 1

To prepare a system for measuring the amount of the PAI.1-tPA, a two antibody sandwich method was examined on various antibody combinations. First, a combination of an anti-PAI.1-MCA as a first antibody and anti-tPA-MCA as a second antibody was examined.

A solution of JTI-1, -2, -3 or -4 with a protein concentration of 10 micrograms/ml was prepared. The solution was coated on a polystyrene plastic well by leaving it to stand overnight at 4 °C on the well. The well was washed, blocked with BSA, and then incubated at 37 °C for 3 hours with a PAI.1-tPA complex in a protein concentration of 0, 10, 20 or 40 ng/ml. After washing, a solution of HRP-labelled JTA-1 in a protein concentration of 2 micrograms/ml was added and incubated at 37 °C for 3 hours. After washing, the peroxidase activity remaining in the well was determined, and the antigen-binding ability of the monoclonal antibody was examined by the sandwich method. The results are shown in Figure 2 (A blank value is substracted in Fig. 2.).

The highest binding ability was obtained when JTI-3 or JTI-4 was used as the first antibody.

EXAMPLE 4

Examination of various antibody combinations 2

A combination of anti-tPA-MCA as a first antibody and anti-PAI.1-MCA as a second antibody was examined.

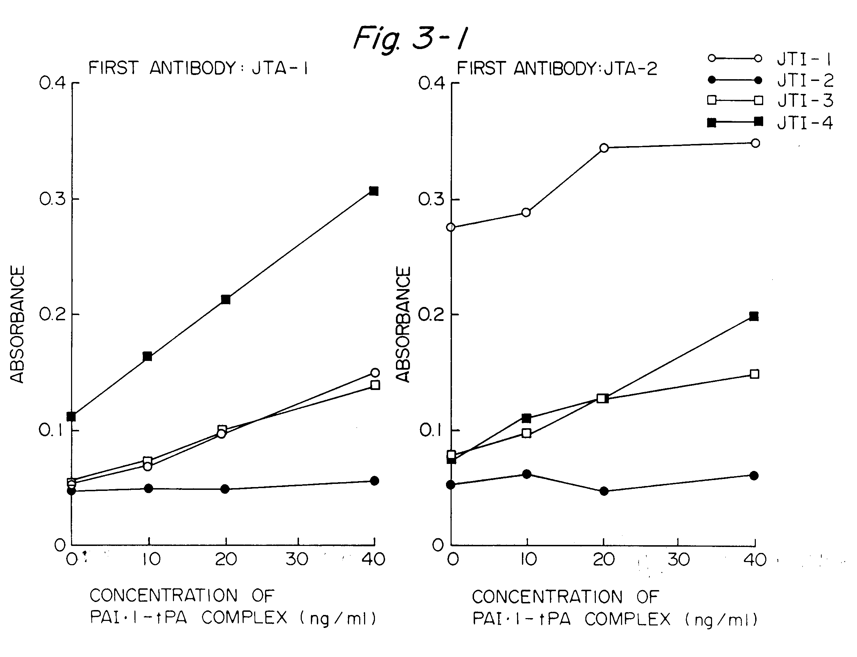

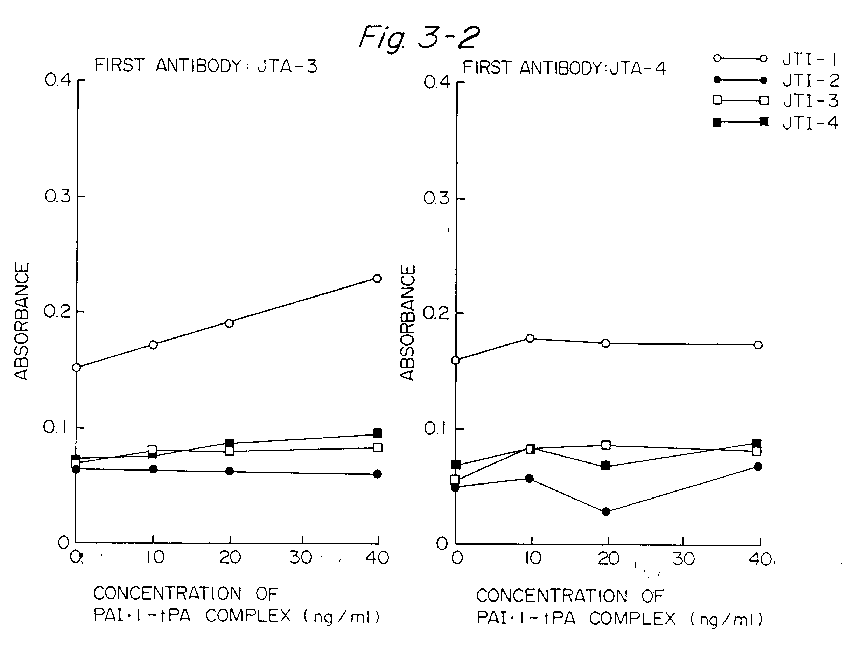

A solution of JTA-1, -2, -3 or -4 having a protein concentration of 10 micrograms/ml was prepared, and coated on a polystyrene plastic well by leaving it to stand overnight at 4 °C on the well. The well was washed, and blocked with BSA. A solution of the PAI.1-tPA complex in a protein concentration of 0, 10, 20 or 40 ng/ml was added and incubated at 37 °C for 3 hours. After washing, a solution of JTI-1, -2, -3 or -4 prepared in a protein concentration of 2 micrograms/ml was added and incubated at 37 °C for 3 hours. After washing, the peroxidase activity remaining in the well was determined, and the antigen-binding ability of the monoclonal antibody was examined by the sandwich method. The results are shown in Figure 3 (A blank value is substracted in Fig. 3.).

The highest binding ability was obtained when JTA-1 was used as the first antibody and JTI-4, as the second antibody.

EXAMPLE 5

Influence of free tPA on the sandwich method involving the use of JTA-1 as a first antibody and JTI-4 as a second antibody

Whether the presence of free tPA in the antibody combination obtained in Example 4 would interfere with the amount of the PAI.1-tPA complex was examined.

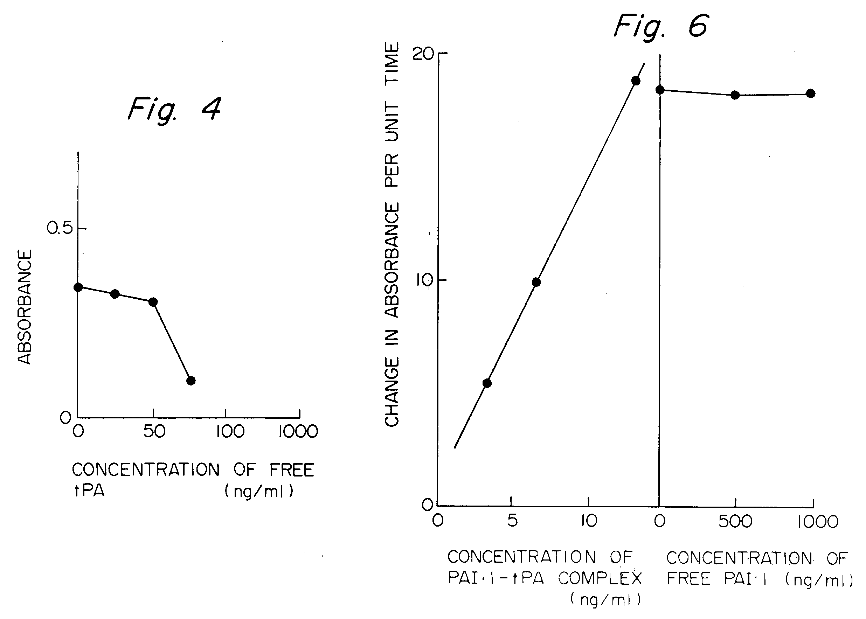

A solution of JTA-1 in a protein concentration of 10 micrograms/ml was coated on a polystyrene plastic well by leaving it to stand overnight at 4 °C on the well. The well was washed, blocked with BSA, and then, incubated at 37 °C for 3 hours with a solution obtained by adding free tPA to a 10 ng/ml solution of the PAI.1-tPA so as to provide a final concentration of 0, 50, 100, or 1,000 ng/ml. After washing, the well was further incubated at 37 °C for 3 hours with a solution of HRP-labelled JTI-4 prepared so as to provide a final concentration of 2 micrograms/ml. After washing, the peroxidase activity remaining in the well was determined, and the degree of interference by free tPA was examined. The results are shown in Figure 4 (A blank value is substracted in Fig. 4.).

The results showed that if the concentration of free tPA in the sandwich system is about 10 times that of the PAI.1-tPA complex, the free tPA interferes with the measured amount of the PAI.1-tPA.

EXAMPLE 6

Influence of free PAI.1 on a sandwich system using JTI-3 or -4 as a first antibody and JTA-1 as a second antibody

Whether the presence of free PAI.1 in the antibody combination obtained in Example 3 would interfere with the amount of the PAI.1-tPA complex was examined.

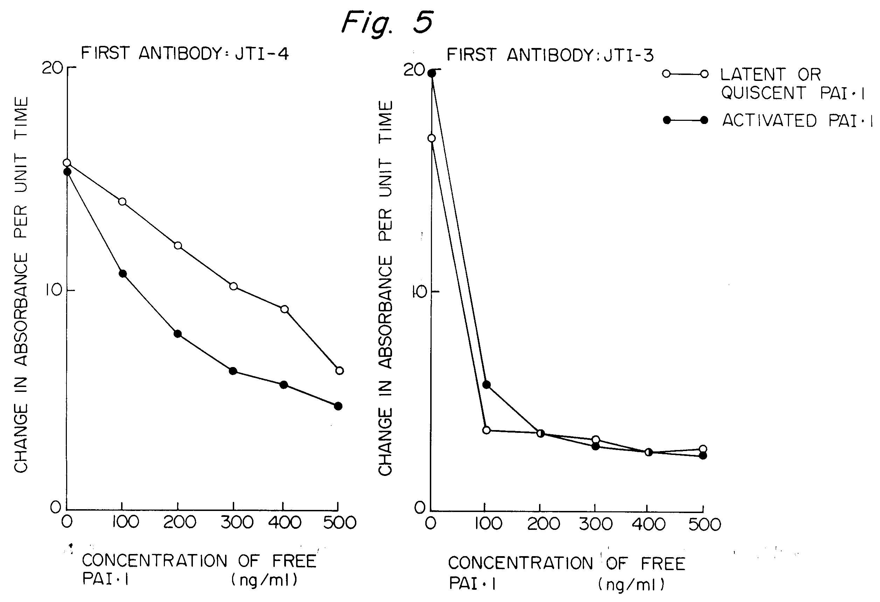

A solution of JTI-3 or JTI-4 in a protein concentration of 10 micrograms/ml was coated on a polystyrene plastic well by leaving it to stand overnight at 4 °C on the well. The well was washed, blcoked with BSA and then, incubated at 37 °C for 3 hours with a solution obtained by adding free PAI.1 to a 10 ng/ml solution of the PAI.1-tPA complex so as to provide a final concentration of 0, 100, 200, 300, 400, or 500 ng/ml. The well was washed, and then incubated at 37 °C for 3 hours with a solution of HRP-labelled JTA-1 in a protein concentration of 2 micrograms/ml. The well was washed, and the peroxidase activity remaining in the well was determined, and the degree of intereference by free PAI.1 was examined. The results are shown in Figure 5 (A blank value is substracted in Fig. 5.).

The results showed that the presence of free PAI.1 in a concentration of about 10 times that of the PAI.1-tPA complex in the sandwich system including JTI-3 or JTI-4 as the first antibody interferes with the amount of the PAI.1-tPA complex determined.

EXAMPLE 7

Influence of free PAI.1 on a sandwich system including JTI-4 as a first antibody bound to beads as a solid carrier and JTA-1 as a second antibody

In the combination of JTL-4 as the first antibody and JTA-1 as the second antibody which showed relatively good results in Example 6, polystyrene beads were used instead of the polystyrene plastic wells in order to increase the amount of the first antibody.

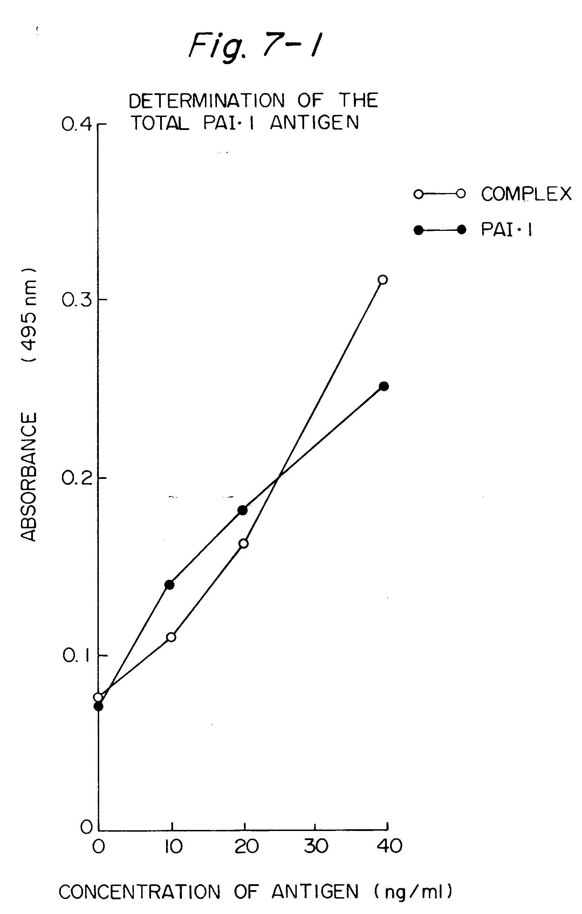

A solution of JTI-4 in a protein concentration was prepared, and polystyrene beads were put in it and left to stand overnight at 4 °C to coat the solution on the beads. The coated beads were washed, put in plastic tubes with one bead for one tube, and blocked with BSA. After washing, a solution obtained by adding free PAI.1 to a 13.2 ng/ml solution of PAI.1-tPA complex so as to provide a final concentration of 0, 500 or 1000 ng/ml was left to stand overnight at 4 °C on the bead. The bead was washed and then incubated at 37 °C for 3 hours with a solution of HRP-labelled JTA-1 prepared in a protein concentration of 2 micrograms/ml. After washing, the peroxidase activity remaining in the bead was determined, and the degree of interference by free PAI.1 was examined. The results are shown in Figure 6 (A blank value is substracted in Fig. 6.).

The results showed that in a system in which the concentration of the first antibody coated is adjusted to 50 micrograms/ml and polystyrene beads are used as the solid carrier, free PAI.1 does not interfere with the amount of the PAI.1-tpA complex determined.

EXAMPLE 8

Determination of total amount of PAI.1 in free PAI.1 and the PAI.1-tPA complex

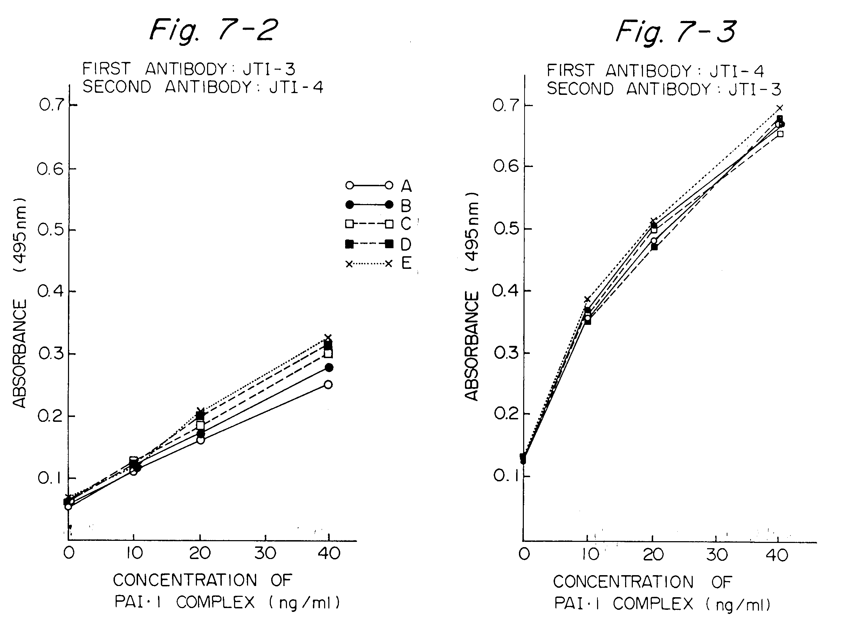

A solution of JTI-3 in a protein concentration of 50 micrograms/ml was coated on a polystyrene plastic well by leaving it to stand overnight at 4 °C on the well. Then, Tris-HCl buffer (TBS for short) containing 1 % BSA was added, and left to stand at 37 °C for 2 hours. Then, the well was washed four times with Tris-HCl buffer containing 0.05 % Tween™ 20 (TBS-Tw for short). A solution of PAI.1 or the PAI.1-tPA complex diluted with TBS-Tw to a protein concentration of 10, 20 or 40 ng/ml was added and left to stand at 37 °C for 2 hours. The well was washed with TBS-Tw four times, and incubated with peroxidase-labelled JTI-4 diluted to 2 micrograms/ml with TBS-Tw. The well was then washed with TBS-Tw four times. A solution of a substrate for peroxidase was added and reacted at room temperature. The absorbance of the solution at a wavelength of 495 nm was then measured. The results are shown in Figure 7-1. A blank value is not substracted in Fig. 7-1. This figure shows that the relation between the protein concentration of PAI.1 alone or the protein concentration of the PAI.1-tPA complex and the absorbance is in a straight line relationship. The above facts show that this assay system permits determination of the total amount of PAI.1.

The above experiment was repeated except that JTI-4 was coated on a polystyrene plastic well and used as the first antibody and peroxidase-labelled JTI-3, as the second antibody. At this time, the mixing ratio between PAI.1 and the PAI.1-tPA complex in the assay sample was varied as shown below in order to determine whether the binding of the antibody to PAI.1 differed from that of the antibody to the PAI.1-tPA complex.

|   | PAI.1 | PAI.1-tPA complex |
|---|-------|-------------------|
| A | 100 % | 0 % |
| B | 70 % | 30 % |
| C | 50 % | 50 % |
| D | 30 % | 70 % |
| E | 9 % | 100 % |

The results are shown in Figures 7-2 and 7-3. A comparison of Figure 7-2 with Figure 7-3 shows that a combination of JTI-4 as the first antibody and JTI-3 as the second antibody has better assay sensitivity. A blank value is not substracted in Fig. 7-2 and Fig. 7-3. Furhermore, there was no substantial difference in the amount of PAI.1 in the PAI.1-tPA complex and the amount of free PAI.1 between the above two antibody combinations, and both antibody combinations permitted exact determination of the total amount of PAI.1.

EXAMPLE 9

Determination of the thrombus dissolution promoting effect in vitro by anti-PAI.1 MCA:-

Quiescent PAI.1 was activated with SDS to give SDS-activated PAI.1. To 100 ng of the SDS-activated PAI.1 was added 10 times or 100 times the amount in moles of activated PAI.1 of JTI-1, -2, -3, -4, mouse IgG or rabbit anti-PAI.1 polyclonal antibody, and the mixture was incubated at 37 °C for 1 hour. Then, 0.1 ng ($1 \times 10^4$ cpm) of tPA labelled with $^{125}$I was added, and the mixture was again incubated at 37 °C. The mixture was subjected to SDS slab gel electrophoresis. The gel was dried and autoradiographed. The detected radioactivity corresponding to tPA alone or the PAI.1-tPA complex was measured, and the radioactivity of tPA alone and that of the PAI.1-tPA complex in percentage are shown in Table 6. As shown in Table 6, when the anti-PAI.1 polyclonal antibody was added in an amount of 100 moles per mole of PAI.1, the formation of the complex was inhibited almost completely. The formation of the complex was also strongly inhibited when JTI-3 was added in a 10-fold or 100-fold amount. JTI-2 also showed a tendency to inhibit the formation of the complex. The above results show that at least JTI-3 can inhibit formation of the PAI.1-tPA complex in vitro.

Table 6

| Inhibition of the formation of PAI.1-tPA complex in vitro | | | | |
|---|---|---|---|---|
| Anitibody | | | tPA | Complex* |
| None | | | 41 % | 59 % |
| Mouse IgG | 100-fold | | 40 | 60 |
| JTI-1 | 10-fold | | 36 | 64 |
| JTI-1 | 100-fold | | 39 | 61 |
| JTI-2 | 10-fold | | 46 | 54 |
| JTI-2 | 100-fold | | 51 | 49 |
| JTI-3 | 10-fold | | 81 | 19 |
| JTI-3 | 100-fold | | 92 | 8 |
| JTI-4 | 10-fold | | 39 | 67 |
| JTI-4 | 100-fold | | 33 | 67 |
| Polyclonal antibody | 100-fold | | 100 | 0 |

\* Complex: PAI.1-tPA complex

EXAMPLE 10

Prevention by anti-PAI.1 MCA of the inhibition of tPA-dependent plasminogen activation by PAI.1

Quiescent PAI.1 was activated with SDS to give SDS-activated PAI.1. To 20 ng of SDS-activated PAI.1 was added JTI-1, -2, -3, -4, rabbit anti-PAI.1 polyclonal antibody in a 10-fold or 100-fold molar amount, and the mixture was incubated at 37 °C for 1 hour. Then, 0.25 ng of tPA was added, and the mixture was incubated at 37 °C for 30 minutes. Plasminogen (22 micrograms) was added, and furthermore, S-2251 as a synthetic coloration substrate for plasmin was added so as to provide a concentration of 0.15 mM. The mixture was allowed to stand at 37 °C for 3 hours, and its absorbance at a wavelength of 405 nm was measured. The amount of SDS-activated PAI.1 was one which inhibited 90 % of the tPA activity. The results are shown in Table 7. Table 7 shows the percentage of the remaining tPA activity in the absence of SDS-activated PAI.1. Table 7 shows that when the anti-PAI.1 polyclonal antibody was added in an amount of 100 moles per mole of PAI.1, the tPA activity was regained almost completely. Furthermore, when JTI-3 was added in an amount of 100 moles, the tPA activity was regained almost completely. The foregoing results show that JTI-3 inhibit the tPA-dependent plasminogen activation inhibition.

Table 7

| Inhibition by anti-PAI.1 MCA of the inhibition of tPA-depending plasminogen activation by PAI.1 | |
|---|---|
| Anitibody | Remaining tPA activity (*) |
| None | 5.9 % |
| Mouse IgG 100-fold | 5.9 |
| JTI-1 10-fold | 7.1 |
| JTI-1 100-fold | 4.7 |
| JTI-2 10-fold | 5.4 |
| JTI-2 100-fold | 4.8 |
| JTI-3 10-fold | 11.9 |
| JTI-3 100-fold | 48.9 |
| JTI-4 10-fold | 5.9 |
| JTI-4 100-fold | 7.1 |
| Polyclonal 10-fold antibody 100-fold | 5.9 95.2 |

(*): The activity measured with tPA alone was taken as 100 %.

EXAMPLE 11

Inhibition by anti-PAI.1 MCA of the formation of the PAI.1-tPA complex

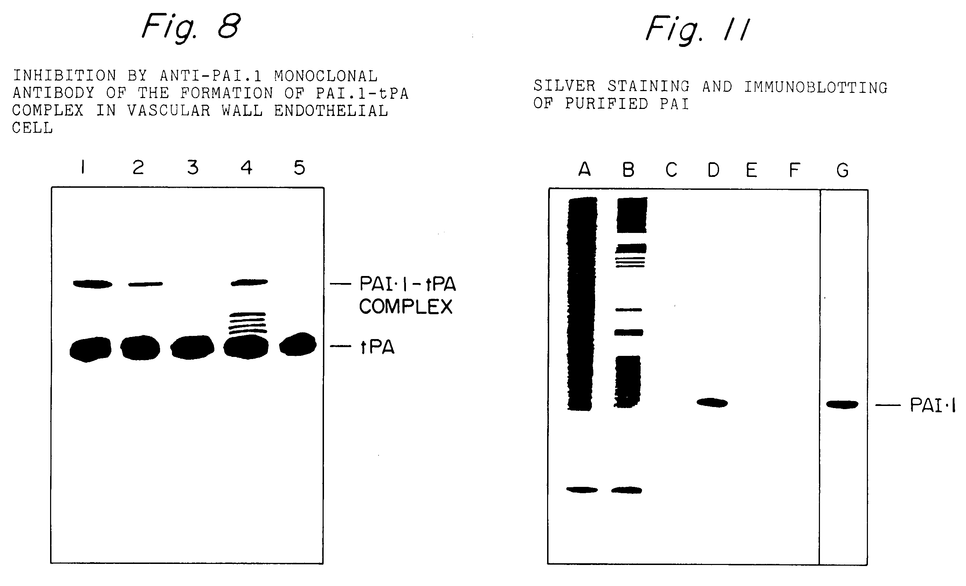

Vascular wall endothelial cells were cultured confluently on a petri dish having a diameter of 35 mm. The cells were then washed with a serum-free 199 medium, and 2 ml of serum-free 199 medium, tPA with a final concentration of 100 ng/ml and JTI-1, -2, -3, -4, mouse IgG or rabbit anti-PAI polyclonal antibody with a final concentration of 10 micrograms/ml were added, and the mixture was left to stand at 37 °C for 3 hours. The supernatant was recovered, and subjected to SDS slab gel electrophoresis. It was then analyzed by fibrin autography. The results are shown in Figure 8. Numerals 1 to 5 in Figure 8 show the following cases.

1: JTI-1 was added
2: JTI-3 was added
3: the polyclonal antibody was added
4: JTI-4 was added
5: tPA used in the experiment

Figure 8 shows that when the rabbit anti-PAI.1 polyclonal antibody was added, the formation of the PAI.1-tPA complex was completely inhibited, and that when JTI-1 or JTI-3 was added, inhibition of the formation of the PAI.1-tPA complex was seen. This shows that JTI-1 and JTI-3 inhibit the formation of the PAI.1-tPA complex by active PAI.1 produced by the vascular wall endothelial cells.

EXAMPLE 12

Fibrinolysis promoting effect by JTI-3

To analyze the fibrinolysis promoting effect of JTI-3, the following experiments were carried out by using the $^{125}$I-fibrin plate method.

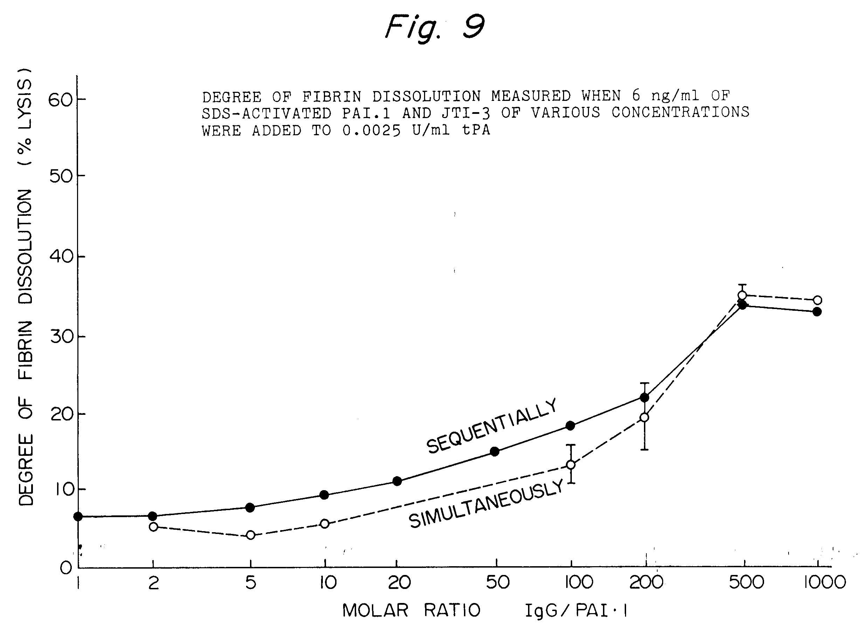

1) SDS-activated PAI.1 (6 ng/ml) and JTI-3 (in an amount corresponding to a PAI.1/JTI-3 mole ratio of from 1/1 to 1/1000) were simultaneously added to a fixed amount of tPA (0.0025 U/ml). The mixture was incubated at 37 °C for 20 minutes, and put in a 24-well polystyrene plate coated with $^{125}$I-fibrin, and at the same time plasminogen was added. The mixture was incubated at 37 °C for 2 hours. The plate was incubated at 37 °C for 2 hours, and the $^{125}$I radioactivity in the supernatant and the $^{125}$I radioactivity remaining on the plate were measured. The degree of dissolution of fibrin (% lysis) was calculated from the ratio of the two. The results are shown in Figure 9 by the curve marked "simultaneously".

2) JTI-3 (in an amount corresponding to a PAI.1/JTI-3 mole ratio of from 1/1 to 1/1000) was added to SDS-activated PAI.1 (6 ng/ml), and the mixture was incubated at 37 °C for 30 minutes. A fixed amount (0.0025 U/ml) of tPA was added, and the mixture was further incubated at 37 °C for 20 minutes. The mixture was put in a 24-well polystyrene plate coated with $^{125}$I-fibrin, and simultaneously, plasminogen was added. The plate was incubated at 37 °C for 2 hours. The $^{125}$I radioactivity in the supernatant and the $^{125}$I radioactivity remaining on the plate were measured, and the degree of fibrin dissolution (% lysis) was calculated from the ratio of the two. The results are shown in Figure 9 by the curve marked "sequentially". A blank value is substracted in Fig. 9.

This experiment shows that even when PAI.1 is present in an amount enough to inhibit the tPA activity almost completely, the addition of JTI-3 inhibits the tPA activity inhibition by PAI.1 depending upon the concentration of JTI-3, and when finally JTI-3 is present in an amount about 500 times the amount of PAI.1, the inhibition of tPA activity by PAI.1 is mostly inhibited.

EXAMPLE 13

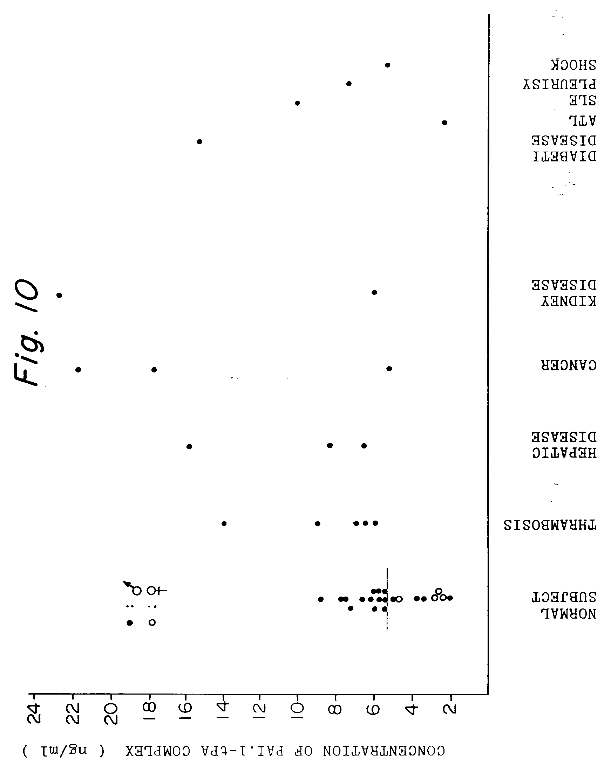

By using a PAI.1-tPA complex assay system of Example 22 described hereinafter, the concentration of the PAI.1-tPA complex in plasma was measured in normal subjects and patients with various diseases (Figure 10). In the normal subjects (21 subjects), the highest concentration of the PAI.1-tPA complex was about 8.8 ng/ml, and the average value of the concentration was 5.3 ng/ml. In contrast, in five patients with thrombosis, the highest concentration was about 14 ng/ml, and the average was 9.5 ng/ml, showing a clear difference from the normal subjects. This shows that the determination of the PAI.1-tPA is helpful in the diagnosis and treatment of thrombosis. In patients with other diseases, a high concentration such as more than 20 ng/ml was observed, and this suggested the possibility of the assay system in accordance with this invention in the diagnosis and therapy of patients with other diseases. A blank value is substracted in Fig. 10.

EP 0 339 302 B1

EXAMPLE 14

Purification of PAI in human vascular wall endothelial cells

One liter of a saturated solution of ammonium sulfate adjusted to a pH of 7.0 was added to 1 liter of a culture supernatant of human vascular wall endothelial cells over 1 hour with ice cooling and stirring. After all the ammonium sulfate solution was added, the mixture was stirred further for 2 hours. The solution was centrifuged at 10,000 x G at 4 °C for 30 minutes, and the precipitate was separated.

The precipitate was dissolved in 100 ml of 20 mM Tris.HCl pH 7.4-0.15 M NaCl-0.01 % Tween™ 80 (Tw 80 for short)-1 mM benzamidine buffer and dialyzed against the same buffer at 4 °C.

The dialyzed sample was mixed with 10 ml of the anti-PAI.1-MCA JTI-1 bound to a carrier in an antibody concentration of 1 mg/ml, and the mixture was stirred at 4 °C for 18 hours. The antibody/carrier product was then washed with 100 ml of 20 mM Tris.HCl-1.0 M NaCl-0.01 % Tw 80-1 mM benzamidine buffer and further with 100 ml of 20 mM Tris-HCl-0.15 M NaCl-0.01 % Tw 80-1 mM benzamidine. PAI.1 was eluted from the antibody/carrier product having PAI.1 adsorbed thereon by using 0.2 M glycine.HCl pH 2.5-0.01 % Tw 80 buffer.

The eluate from the column was fractionated into 2 ml fractions. After the elution, 1 M Tris pH 9.0 was added to the fractions to adjust their pH to 7.4. To remove the PAI.1-tPA complex present in the resulting sample, anti-tPA polyclonal antibody was stirred with an insoluble carrier at 4 °C for 18 hours, and a supernatant was obtained.

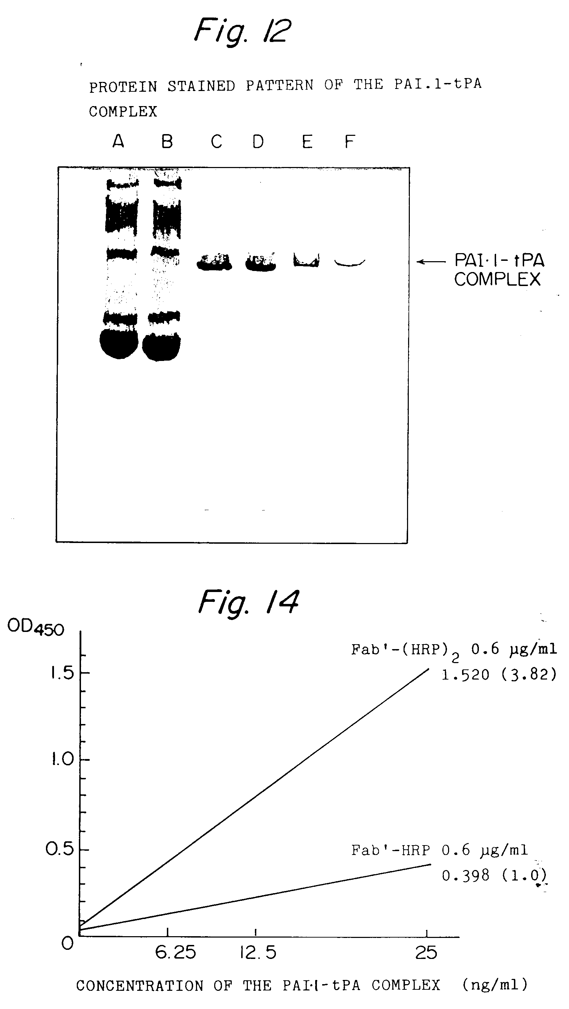

To determine the purity of PAI.1, the supernatant was subjected to SDS electrophoresis. The results of protein staining and immunoblotting with anti-PAI.1 MCA, JTI-2 and HRP-labelled rabbit anti-mouse IgG are shown in Figure 11.

In Figure 11, the symbols A to F show the colors of silver staining of the following.

A:    culture supernatant
B:    the fraction which passed through the antibody column without adsorption
C:    fraction 1
D:    fraction 2
E:    fraction 3
F:    fraction 4
      G shows immunoblotting coloration.

The same experiment except using JTI-3 bound to carrier instead of JTI-1 bound to carrier was carried out. Result was almost the same as above.

EXAMPLE 15

Purification of PAI.1-tPA complex

Human vascular wall endothelial cells were cultured in a monolayer, and washed with serum-free 199 medium. The serum-free 199 medium, tPA in a final protein concentration of 1 microgram/ml and ultrosera G (a product of LKB) in a final concentration of 3 % were added, and the mixture was incubated overnight. The supernatant was recovered, and a proteinase inhibitor was added. The mixture was incubated overnight at 4 °C. The resin was washed and the substance bound to it was eluted with 0.2 M glycine.HCl (pH 2.5) under acidic conditions. The eluted fraction was neutralized and further incubated overnight at 4 °C with Sepharose™ 4B resin to which anti-tPA polyclonal antibody was fixed. Figure 12 shows the silver stained pattern of the resulting fraction. It is seen from this figure that a single PAI.1-tPA complex was obtained.

Similar results were obtained when the above procedure was repeated using JTA-1 instead of the anti-tPA polyclonal antibody.

EXAMPLE 16

Preparation of an enzyme-labelled antibody

A Fab' fragment of a monoclonal antibody (JTA-1) against human tissue plasminogen activator was prepared in accordance with the method of Nisonoff, and bound to peroxidase in a customary manner to obtain peroxidase-labelled Fab' ["Biochemical Experiment Lectures (continued)" edited by Japan Biochemical Society, Vol. 5, pages 109-112, 1988 published by Tokyo Kagaku Dojin]. Specifically, 40 micrograms of pepsin was added to 2 ml of a 1.0 mg/ml solution of the monoclonal antibody (0.01 M PBS, pH 7.2), and the

23

solution was adjusted to pH 3.7 with 1 M citrate buffer (pH 3.5). Thereafter, the solution was maintained at 37 °C for 1 hour to perform digestion. IM(IN)-NaOH was added dropwise to the reaction solution to adjust the pH to 8 and stop the reaction. F(ab')$_2$ fragment having a molecular weight of 100,000 was separated from the reaction solution by HPLC on a TSK Gel G-3000SW column using 5 mM EDTA-0.1 M phosphate buffer as an eluent.

It was concentrated by means of an ultrafilter. 200 microliters of 0.1 M 2-mercaptoethylamine was added, and the mixture was subjected to reducing treatment at 37 °C for 2 hours. The reaction solution was concentrated by an ultrafilter and subjected to HPLC in the same way as in the separation of F(ab')$_2$ to separate Fab' fragment having a molecular weight of 50,000 and give 1.0 ml of a solution of Fab' fragment (1.07 mg/ml).

Separately, 6.0 mg of horseradish peroxidase (a product of Toyobo) was dissolved in 0.9 ml of 0.1 M sodium phosphate buffer (pH 7.0). 60 microliters (2.9 mg/80 microliters) of a dimethylformamide solution of N-succinimidyl-3-maleimidemethyl-cyclohexanecarbonate was added dropwise, and the reaction was carried out at 30 °C for 1 hour. The reaction solution was charged onto a Sephadex™ G-25 column. 0.1 M sodium phosphate buffer (pH 6.0) was passed through the column to separate maleimidized peroxidase. Two hundred microliters of the resulting maleimide-peroxidase (5.28 mg/ml) was added dropwise to the Fab' fragment solution obtained as described above, and they were reacted at 4 °C for 20 hours. The reaction solution was concentrated by an ultrafilter and subjected to HPLC on a TSK Gel G-3000SW column using PBS (pH 7.2) as an eluent to give peroxidase-labelled Fab' having a molecular weight of 90,000. The labelled Fab' was used in the preparation of a calibration curve described below.

Preparation of a solid antibody

Monoclonal antibody (JTI-4) to anti-human Pal.1 was dissolved in 0.1 M phosphate-citrate buffer (pH 3.0) in a concentration of 20 micrograms/ml, and the solution was distributed in a microplate at a rate of 200 microliters per well, and left to stand for one day at 4 °C to immobilize the antibody to the plate. The plate was then washed with PBS, and 1.0 % BSA-PBS was added at a rate of 250 microliters and left to stand at 4 °C for one day for after coating. The plate was washed with PBS to prepare an antibody-immobilized microplate.

Preparation of a calibration curve

A dilution series of the PAI.1-tPA complex in a concentration of 0, 0.3, 0.6, 1.25, 5 and 10 ng/ml was prepared using skimmed milk with a final concentration of 0.25 %/10 mM phosphate/0.5 M NaCl (pH 7.2), and added to the antibody-bound plate at a rate of 200 microliters per well and reacted at room temperature for 2 hours. The reaction mixture was washed with a washing buffer composed of 10 mM phosphate, 0.5 M NaCl and 0.05 % Tween 20, and then diluted with the washing buffer so that the concentration of the peroxidase-labelled Fab' component was 0.3 microgram/ml. The labelled Fab' solution was distributed at a rate of 200 microliters per well, and reacted at room temperature for 1 hour. The reaction mixture was washed with the washing buffer, and 200 microliters of a solution of a substrate for peroxidase (containing 2.5 mM H$_2$O$_2$, 0.025 % 3,3',5,5'-tetra-methylbenzidine) was added and colored at room temperature for 1 hour. An 8N-sulfuric acid solution (25 microliters) was added to perform a stopping reaction, and the absorbance at 450 nm was measured by a microplate reader.

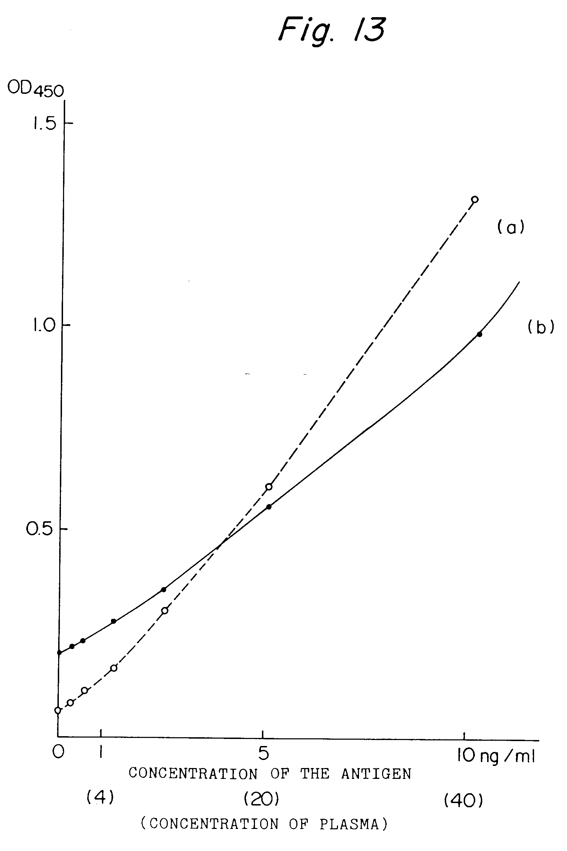

The resultng calibration curve is shown as (a) in Figure 13. From this curve, it can be seen that the lower limit of measurement was 0.3 ng/ml.

In the above procedure of Example 15, an enzymelabelled antibody was prepared by using anti-human tPA monoclonal antibody (JTA-1) instead of the Fab' fragment in accordance with the method of Nakane et al. (P. K. Nakane et al., J. Histochem. Cytochem., 22, 1084, 1974). A calibration curve was prepared under the same conditions as in Example 16 (the above labelled antibody was adjusted to 0.6 microgram/ml as the concentration of IgG so that it became the same as in Example 16).

The resulting calibration curve is shown in (b) in Figure 13.

It is seen from Figure 13 that when the Fab' fragment-HRP was used as the labelled antibody [Example 16, (a)], non-specific adsorption was less than in the case of using the whole antibody-HRP as the labelled antibody.

EXAMPLE 17

Preparation of an enzyme labelled antibody composed of the Fab' fragment and peroxidase in a ratio of 1:2

The Fab' fragment (0.1 mg) obtained in Example 16 and 3.2 mg of maleimidized peroxidase were reacted at 25 °C for 24 hours and the reaction product was separated and purified by HPLC on a TSK Gel 3000SW using 0.01 M PBS as an eluent to give 0.35 mg of a labelled antibody having a molecular weight of about 130,000. The mole ratio of the Fab' fragment to peroxidase was 1:2 from the absorbances of the labelled antibodies at 280 nm and 403 nm. This labelled antibody will be abbreviated hereinafter as Fab'-$(HRP)_2$.

EXAMPLE 18

Preparation of a immobilized antibody

Polystyrene beads (6.35 mm in diameter, #80, a product of Sekiksui Chemical Co., Ltd.) were put in a 0.1 M phosphate buffer solution (pH 9.0) of PAI.1-MCA (JTI-4) (20 micrograms/ml), and the solution was left to stand at 4 °C for 20 hours. The beads were washed three times with 0.01 M PBS and left to stand at room temperature for 2 hours in a 1 % BSA-PBS solution and again washed three times with 0.01 M PBS to give a immobilized antibody.

Preparation of a calibration curve

The human PAI.1-tPA was diluted with 0.01 M phosphate-0.5 M NaCl buffer (pH 7.2) containing skimmed milk having a final concentration of 0.25 % to prepare a standard solution having a concentration of 25, 12.5, 6.25, 3.125, or 0 ng/ml. The standard solution (0.3 ml) and the immobilized beads were put in a small test tube, and incubated at 37 °C for 2 hours. The beads were washed three times with 0.01 M PBS-0.05 % Tween 20 (pH 7.2). Then, 0.3 ml of the Fab'-HRP obtained in Example 16 and the Fab'-$(HRP)_2$ obtained in Example 17 diluted with 0.01 M PBS-0.05 % Tween 20 (pH 7.2) to a Fab' fragment concentration of 0.6 microgram/ml was added and incubated at 37 °C for 30 minutes. The beads were washed three times with 0.01 M PBS-0.05 % Tween 20, and a solution of a substrate for peroxidase (containing 2.5 mM $H_2O_2$-0.025 % 3,3',5',5'-tetramethylbenzidine) was added and coloration reaction was carried out at 37 °C for 30 minutes. The coloration reaction was stopped by adding IN-sulfuric acid, and the absorbance at 450 nm was measured.

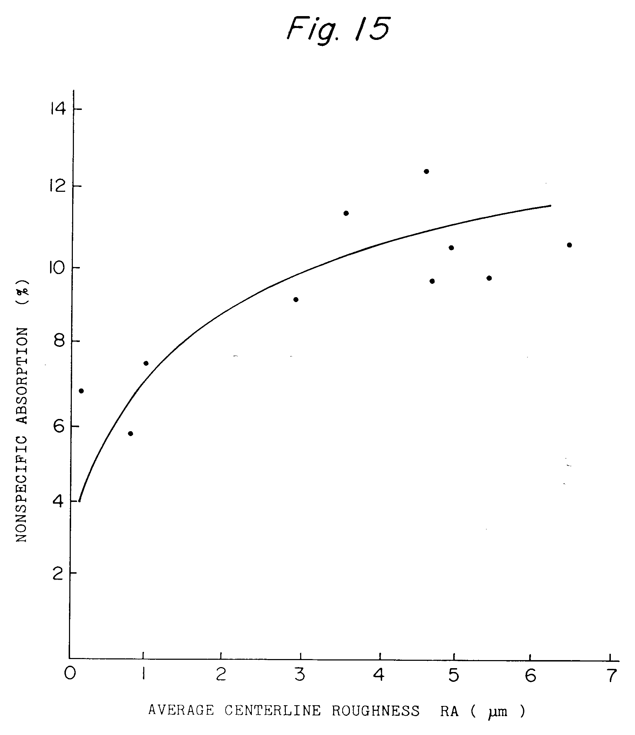

The resulting calibration curve is shown in Figure 14.

Figure 14 shows that the peroxidase-labelled antibody Fab'-$(HRP)_2$ obtained in Example 17 showed about 4 times as high activity as the peroxidase-labelled antbody (Fab'-HRP) obtained in Example 16.

EXAMPLE 19

Beads of different surface roughnesses were used as polystyrene beads for EIA, and the antibody was immobilized thereto as in Example 18. The absorbances were measured as in Example 18 except that a standard solution (Std) having a concentration of 25 nm/ml and Fab'-$(HRP)_2$ as the labelled antibody were used.

Figure 15 shows the percentage of non-specific adsorption given by the following equation.

$$\text{Percent non-specific adsorption (\%)} = \frac{\text{Absorbance of Std (0 ng/ml)}}{\text{Absorbance of Std (2.5 ng/ml)}}$$

It is seen from Figure 15 that when polystyrene beads having an average roughness (Ra), measured by a surface roughness tester (SURFCOM made by Tokyo Seimitsu K. K.), of not more than 1.5 micrometers were used, non-specific adsorption was low, and they were effective for the assay in accordance with this invention.

REFERENTIAL EXAMPLE 1

Preparation of a Fab' fragment of mouse monoclonal antibody

A 1 M citrate buffer (pH 1.5) was added little by little to 10 ml of a 0.01 M PBS solution (1 mg/ml) of mouse anti-human tPA antibody (JTA-1) to adjust the pH of the PBS solution to 3.75. To the resulting solution was added 0.2 ml of a solution of pepsin in citrate buffer (1 mg/ml), and the mixture was incubated at 37 °C for 1 hour. The reaction solution was adjusted to pH about 6.0 with IN-NaOH, and concentrated to about 1 ml by an ultra-filter (Filtron omega cell purchased from by Fuji Filter Co., Ltd.), and subjected to HPLC on a TSK Gel G-3000SW column (Toso) using 0.1 M phosphate buffer (pH 6.0) containing 5 mM EDTA as an eluent to isolate 5.9 mg of a F(ab')$_2$ fragment.

To 2.0 ml of 0.1 M phosphate buffer containing 5.9 mg of the F(ab')$_2$ fragment was added 0.2 ml of a solution of 0.1 M 2-mercaptoethylamine in phosphate buffer, and the mixture was incubated at 37 °C for about 90 minutes. The reaction product was subjected to HPLC under the same conditions as above to obtain 5.3 mg of a Fab' fragment.

REFERENTIAL EXAMPLE 2

Preparation of maleimidized peroxidase

0.6 ml of a dimethylformamide solution of succinimidyl 4-(N-maleimidemethyl)cyclohexanecarbonate (33.4 mg/ml) was added to 6 ml of a solution of 40 mg of peroxidase (Toyobo) in 0.1 M phoaphste buffer (pH 7.0), and the mixture was stirred at 30 °C for 1 hour. The reaction mixture was centrifuged and then subjected to gel filtration on a Sephadex G-25 column using 0.1 M phosphate buffer (pH 7.0) as an eluent to give 39 mg of maleimidized peroxidase.

EXAMPLE 20

Preparation of an enzyme-labelled antibody composed of Fab' fragment and peoxidase in a ratio of 1:2

The Fab' fragment (1.0 mg) obtained in Referential Eaxmple 1 and the maleimidized peroxidase (6.4 mg) obtained in Referential Example 2 were reacted at 25 °C for 24 hours, and the product was purified by HPLC on a TSK Gel G-3000SW column using 0.01 M PBS as an eluent to give 0.53 mg of a labelled antibody having a molecular weight of about 130,000. From the absorbances of the resulting labelled antibody at 280 nm and 403 nm, it was found that the binding ratio of the Fab' fragment to peroxidase was 1:2. This labelled antibody will be abbreviated hereinafter as Fab'-(HRP)$_2$.

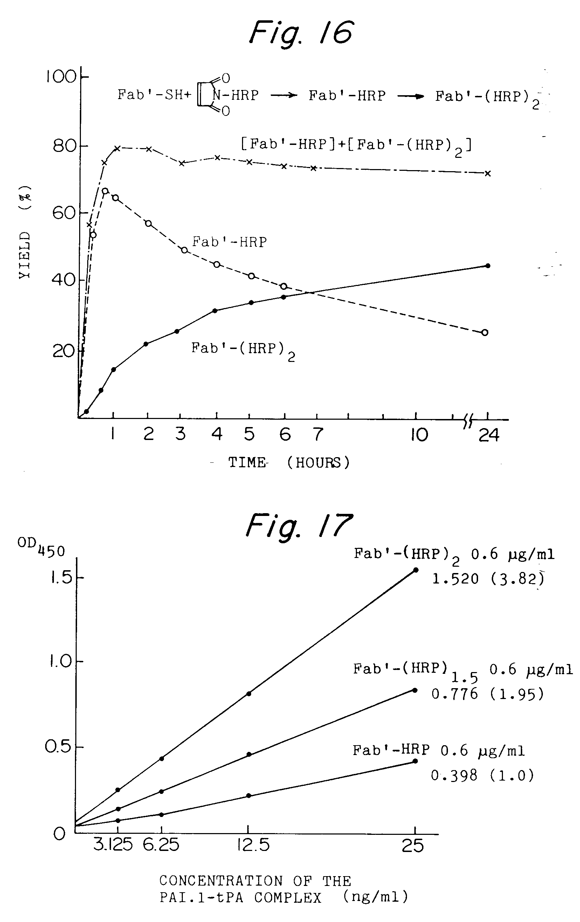

The reaction yield of the labelled antibody with time was as shown in Figure 16.

The above reaction was carried out in the same way except that as shown in Table 8, the mole ratio between the Fab' fragment and the maleimidized peroxidase and the reaction temperature were changed. The yield of the labelled antibody was as shown in Table 8.

Table 8

| Reaction temperature (°C) | Reaction time (hours) | Fab'/maleimidized HRP ratio | Yield (%) of Fab'-(HRP)$_2$ | Yield (%) of Fab'-HRP |
|---|---|---|---|---|
| 4 | 24 | 1:1 | 3.2 | 61.0 |
| 30 | 24 | 1:1 | 15.1 | 52.0 |
| 25 | 24 | 1:4 | 35.0 | 30.3 |
| 25 | 24 | 1:8 | 53.4 | 32.0 |
| 25 | 24 | 1:20 | 55.3 | 33.1 |
| 45 | 24 | 1:4 | 55.8 | 30.8 |

EXAMPLE 21

(1) Preparation of an enzyme-labelled antibody composed of Fab' fragment and peroxidase in a ratio of 1:1.5

Fab' fragment and maleimidized peroxidase were reacted under the same conditions as in Example 20, and the reaction mixture was separated on a column of Ultrogel AcA44 (LKB) using 0.1 M phosphate buffer as an eluent. From the absorbances of the resulting labelled antibody at 280 nm and 403 nm, it was found that the binding ratio of the Fab' fragment to peroxidase was 1:1.5. This labelled antibody will be abbreviated hereinafter as Fab'-(HRP)$_{1.5}$.

(2) Preparation of an enzyme-labelled antibody composed of Fab' fragment and peroxidase in a ratio of 1:1

The Fab' fragment (1.0 mg) obtained in Referential Example 1 and the maleimidized peroxidase (0.8 mg) obtained in Referential Example 2 were reacted at 4 °C for 24 hours and purified by HPLC to give 0.61 mg (as Fab') of an enzyme-labelled antibody having a molecular weight of about 90,000 in which the Fab' fragment and the peroxidase were bound in a ratio of 1:1. This enzyme-labelled antibody will be abbreviated hereinafter as Fab'-HRP.

(3) Preparation of a calibration curve

The PAI.1-tPA complex was diluted with 0.01 M phosphate-0.5 M NaCl buffer (pH 7.2) containing 0.25 % of skimmed milk to prepare a standard solution having a concentration of 25, 12.5, 6.25, 3.125 or 0 ng/ml. 0.3 ml of the standard solution and the immobilized beads were put in a small test tube and incubated at 37 °C for 2 hours, and washed three times with 0.01 M PBS-0.05 % Tween 20 (pH 7.2). The peroxidase-labelled Fab' fragments obtained in Example 20 Fab'-(HRP)$_2$ and Example 21, (1) Fab'-(HRP)$_{1.5}$ and (2) Fab'-HRP were each diluted with 0.01 M PBS-0.05 % Tween 20 (pH 7.2) so that the Fab' fragment concentration became 0.6 microgram/ml. The labelled antibody solution (0.3 ml) was added and incubated at 37 °C for 30 minutes. The bead was washed three times with 0.01 M PBS-0.05 % Tween 20, and 0.3 ml of a solution of a substrate for peroxidase (containing 2.5 mM H$_2$O$_2$-0.025 % 3,3',5,5'-tetramethylbenzidine) was added to permit coloration reaction at 37 °C for 30 minutes. The reaction was stopped by adding IN-sulfuric acid, and the absorbance at 450 nm was measured.

The resulting calibration curves are shown in Figure 17. It is seen from Figure 17 that the peroxidase-multilabelled antibody obtained in Example 20 has about 4 times as high activity as peroxidase-labelled antibody (Fab'-HRP) obtained in Example 21, (2), and that the multilabelled antibody obtained in Example 21, (1) has about twice as high activity as the Fab'-HRP.

EXAMPLE 22

Use of specular polystyrene bead

PAI.1 MCA (JTI-4) was immobilized on specular polystyrene beads (D-7, Ra = 0.85 nm, made by Immuno-chemical: Okayama, Japan) and assayed by using the peroxidase-labelled antibody Fab'-(HRP)$_2$ as in Example 18.

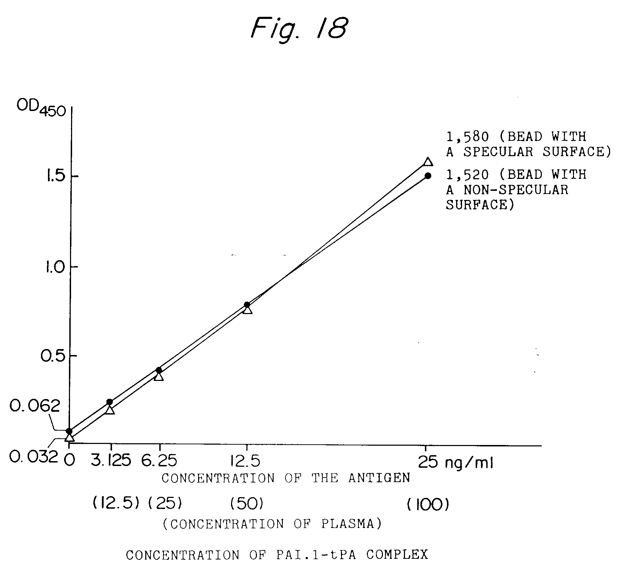

Figure 18 shows that the non-specific binding of labelled antibody on the specular bead is about half of that on a non-specular polystyrene bead (#80, a product of Sekisui Chemical Co., Ltd.).

PAI-tPA complex concentration in plasma samples of normal human subjects are summarized in Table 9.

## Table 9

| No. | Results (ng/ml) | No. | Results (ng/ml) |
|-----|-----------------|-----|-----------------|
| 1 | 3.6 | 13 | 5.4 |
| 2 | 5.0 | 14 | 7.3 |
| 3 | 6.2 | 15 | 5.8 |
| 4 | 7.7 | 16 | 8.8 |
| 5 | 6.0 | 17 | 5.8 |
| 6 | 2.1 | 18 | 2.8 |
| 7 | 6.0 | 19 | 2.5 |
| 8 | 5.5 | 20 | 4.7 |
| 9 | 5.5 | 21 | 2.7 |
| 10 | 3.5 | | |
| 11 | 6.6 | average | 5.3 |
| | | SD | |
| 12 | 7.5 | | 1.8 |

**Claims**

1.  Monoclonal antibody JTI-3 against human plasminogen activator inhibitor 1 which was produced by hybridoma JTI-3 deposited as deposit No. BP-2317 at Fermentation Research Institute, Japan.

2.  Monoclonal antibody JTI-4 against human plasminogen activator inhibitor 1 which was produced by hybridoma JTI-4 deposited as deposit No. BP-2318 at Fermentation Research Institute, Japan.

3.  Monoclonal antibody JTA-1 against human plasminogen activator which was produced by hybridoma JTA-1 deposited as deposit No. BP-2316 at Fermentation Research Institute, Japan.

4.  A method of immunologically assaying a human plasminogen activator inhibitor 1-human tissue plasminogen activator complex in a human assay sample which comprises
    (i) bringing the human assay sample into contact with a first antibody bound to an insoluble solid carrier, then washing the carrier and bringing it into contact with a labelled second antibody, or
    (ii) bringing the first antibody bound to the insoluble solid carrier, the labelled second antibody and the human assay sample into contact with one another in the same assay system,
    wherein one of the first and second antibodies is the monoclonal antibody JTI-3 of claim 1 or JTI-4 of claim 2 against human plasminogen activator inhibitor 1 or an equivalent fragment thereof, and the other is the monoclonal antibody JTA-1 of claim 3 against human tissue plasminogen activator or an equivalent fragment thereof.

5.  A method of immunologically assaying a human plasminogen activator inhibitor 1-human tissue plasminogen activator complex in a human assay sample which comprises

(i) bringing the human assay sample into contact with a first antibody bound to an insoluble solid carrier, then washing the carrier and bringing it into contact with a labelled second antibody, or
(ii) bringing the first antibody bound to the insoluble solid carrier, the labelled second antibody and the human assay sample into contact with one another in the same assay system,
wherein one of the antibodies is the monoclonal antibody JTI-3 of claim 1 or JTI-4 of claim 2 against human plasminogen activator inhibitor 1, and the other is the monoclonal antibody JTA-1 of claim 3 against human tissue plasminogen activator, and labelled second antibody is a labelled Fab' fragment of a monoclonal antibody.

6. The method of claim 5 in which the labelled second antibody is an enzyme-labelled Fab' fragment of a monoclonal antibody.

7. The method of claim 5 in which the labelled second antibody is a multilabelled antibody comprising a Fab' fragment of an antibody and at least 1.5 molecules on an average, per molecule of the Fab' fragment, of an enzyme bound thereto via the sulfur atoms of the Fab' fragment.

8. The method of claim 5 in which the insoluble solid carrier is a polystyrene bead whose surface is rendered specular.

9. The method of claim 5 in which the immunological reaction is carried out in the presence of a non-specific adsorption inhibitor.

10. The method of claim 9 in which the non-specific adsorption inhibitor is at least one protein having a molecular weight of about 16,000 to about 50,000 and an isoelectric point of 1.0 to 5.0, and the final concentration of the protein in the immunological reaction solution is 0.002 to 0.9 % by weight.

11. The method of claim 10 in which the immunological reaction is carried out in the presence of skimmed milk, and the final concentration of the skimmed milk in the immunological reaction solution is adjusted to 0.002 to 0.8 % by weight.

12. A reagent system for immunologically assaying a human plasminogen activator inhibitor 1-human tissue plasminogen activator complex in a human assay sample by using a first antibody bound to an insoluble solid carrier and a labelled second antibody, one of the antibodies being the monoclonal antibody JTI-3 of claim 1 or JTI-4 of claim 2 against the human plasminogen activator inhibitor 1 or an equivalent fragment thereof and the other being the monoclonal antibody JTA-1 of claim 3 against the human tissue plasminogen activator or an equivalent fragment thereof.

13. The reagent system of claim 12 in which the first antibody is the monoclonal antibody JTI-3 or JTI-4 against human plasminogen activator inhibitor 1 or an equivalent fragment thereof, and the second antibody is the monoclonal antibody JTA-1 against human tissue plasminogen activator or an equivalent fragment thereof.

14. The reagent system of claim 12 or 13 in which the monoclonal antibody to human plasminogen activator inhibitor 1 is the monoclonal antibody JTI-4.

15. The reagent system of any one of claims 12 to 14 in which the insoluble solid carrier is a plastic receptacle, a plastic bead, a plastic stick, a latex bead, a glass bead or a metal particle.

16. The reagent system of any one of claims 12 to 15 in which the labelled second antibody is an antibody labelled with an enzyme, a radioisotope, a luminescent substance or a fluorescent substance.

17. The reagent system of claim 12 in which in the labelled second antibody, the antibody is a Fab' fragment of a monoclonal antibody.

18. The reagent system of claim 12 in which the labelled second antibody is a Fab' fragment of a monoclonal antibody, which is labelled with an enzyme.

**19.** The reagent system of claim 18 in which the labelled second antibody is a multilabelled antibody comprising a Fab' fragment of an antibody and at least 1.5 molecules on an average, per molecule of the Fab, fragment, of an enzyme bound thereto through the sulfur atoms of the Fab' fragment.

**20.** The reagent system of claim 12 in which the insoluble solid carrier is a polystyrene bead whose surface is rendered specular.

**21.** A kit for immunologically assaying a human plasminogen activator inhibitor 1-human tissue plasminogen activator complex in a human assay sample, comprising a combination of

(i) a reagent system of claim 12,

(ii) a dissolving agent,

(iii) a washing agent, and

(iv) where an antibody labelled with an enzyme is used, a substrate for measuring the enzyme activity and a reaction stopper,

one of the antibodies being monoclonal antibody JTI-3 of claim 1 or JTI-4 of claim 2 against human plasminogen activator inhibitor 1 or an equivalent fragment thereof and the other being monoclonal antibody JTA-1 of claim 3 against human tissue plasminogen activator.

**22.** A method of diagnosing the condition of thrombosis, which comprises determining a human plasminogen activator inhibitor 1-human tissue plasminogen activator complex in a human assay sample by the method of claim 4, and analyzing the determined value.

**Patentansprüche**

**1.** Monoclonaler Antikörper JTI-3 gegen den menschlichen Plasminogen-Aktivator-Inhibitor 1, produziert von dem Hybridom JTI-3, hinterlegt unter der Hinterlegungsnummer BP-2317 bei dem Fermentation Research Institute, Japan.

**2.** Monoclonaler Antikörper JTI-4 gegen menschlichen Plasminogen-Aktivator-Inhibitor 1, produziert von dem Hybridom JTI-4, hinterlegt unter der Hinterlegungsnummer BP-2318 bei dem Fermentation Research Institute, Japan.

**3.** Monoclonaler Antikörper JTA-1 gegen menschlichen Plasminogen-Aktivator, produziert von dem Hybridom JTA-1, hinterlegt unter der Hinterlegungsnummer BP-2316 bei dem Fermentation Research Institute, Japan.

**4.** Verfahren zum immunologischen Test auf einen menschlichen Plasminogen-Aktivator-Inhibitor 1-menschlichen Gewebsplasminogen-Aktivator-Komplex in einer menschlichen Testprobe, wobei man

(i) die menschliche Testprobe in Kontakt mit einem ersten Antikörper, der an einen unlöslichen festen Träger gebunden ist, bringt, dann den Träger wäscht und ihn in Kontakt mit einem markierten zweiten Antikörper bringt, oder

(ii) den ersten Antikörper, der an den unlöslichen festen Träger gebunden ist, den markierten zweiten Antikörper und die menschliche Testprobe in Kontakt miteinander in dem gleichen Assaysystem bringt,

dadurch **gekennzeichnet,** daß entweder der erste oder der zweite Antikörper der monoclonale Antikörper JTI-3 nach Anspruch 1 oder JTI-4 nach Anspruch 2 gegen den menschlichen Plasminogen-Aktivator-Inhibitor 1 oder ein äquivalentes Fragment davon ist, und der andere der monoclonale Antikörper JTA-1 nach Anspruch 3 gegen menschlichen Gewebsplasminogen-Aktivator oder ein äquivalentes Fragment davon ist.

**5.** Verfahren zum immunologischen Test auf einen menschlichen Plasminogen-Aktivator-Inhibitor 1-menschlichen Gewebsplasminogen-Aktivator-Komplex in einer menschlichen Testprobe, wobei man

(i) die menschliche Testprobe in Kontakt mit einem ersten Antikörper, der an einen unlöslichen festen Träger gebunden ist, bringt, dann den Träger wäscht und ihn in Kontakt mit einem markierten zweiten Antikörper bringt, oder

(ii) den ersten Antikörper, der an den unlöslichen festen Träger gebunden ist, den markierten zweiten Antikörper und die menschliche Testprobe in Kontakt miteinander in dem gleichen Assaysystem bringt,

dadurch **gekennzeichnet,** daß einer der Antikörper der monoclonale Antikörper JTI-3 nach Anspruch 1 oder JTI-4 nach Anspruch 2 gegen menschlichen Plasminogen-Aktivator-Inhibitor1 ist, und der andere der monoclonale Antikörper JTA-1 nach Anspruch 3 gegen menschlichen Gewebsplasminogen-Aktivator ist, und der markierte zweite Antikörper ein markiertes Fab'-Fragment eines monoclonalen Antikörpers ist.

6. Verfahren nach Anspruch 5, dadurch **gekennzeichnet,** daß der markierte zweite Antikörper ein Enzymmarkiertes Fab'-Fragment eines monoclonalen Antikörpers ist.

7. Verfahren nach Anspruch 5, dadurch **gekennzeichnet,** daß der markierte zweite Antikörper ein mehrfach markierter Antikörper, umfassend ein Fab'-Fragment eines Antikörpers und mindestens durchschnittlich 1,5 Moleküle pro Molekül des Fab'-Fragments eines über das Schwefelatom des Fab'-Fragments daran gebundenen Enzyms ist.

8. Verfahren nach Anspruch 5, dadurch **gekennzeichnet,** daß der unlösliche feste Träger eine Polystyrolkugel ist, dessen Oberfläche glatt gemacht wurde.

9. Verfahren nach Anspruch 5, dadurch **gekennzeichnet,** daß die immunologische Reaktion in Gegenwart eines nicht-spezifischen Adsorptionsinhibitors durchgeführt wird.

10. Verfahren nach Anspruch 9, dadurch **gekennzeichnet,** daß der nicht-spezifische Adsorptionsinhibitor mindestens ein Protein mit einem Molekulargewicht von etwa 16.000 bis etwa 50.000 und einem isoelektrischen Punkt von 1,0 bis 5,0 ist, und die Endkonzentration des Proteins in der immunologischen Reaktionslösung 0,002 bis 0,9 Gew.-% beträgt.

11. Verfahren nach Anspruch 10, dadurch **gekennzeichnet,** daß die immunologische Reaktion in Gegenwart von Magermilch durchgeführt wird, und daß die Endkonzentration der Magermilch in der immunologischen Reaktionslösung auf 0,002 bis 0,8 Gew.-% eingestellt wird.

12. Reagenssystem zum immunologischen Assay auf einen menschlichen Plasminogen-Aktivator-Inhibitor 1-menschlichen Gewebsplasminogen-Aktivator-Komplex in einer menschlichen Testprobe unter Verwendung eines ersten Antikörpers, der an einen unlöslichen festen Träger gebunden ist, und eines markierten zweiten Antikörpers, wobei einer der Antikörper der monoclonale Antikörper JTI-3 nach Anspruch 1 oder JTI-4 nach Anspruch 2 gegen den menschlichen Plasminogen-Aktivator-Inhibitor 1 oder ein äquivalentes Fragment davon ist, und der andere der monoclonale Antikörper JTA-1 nach Anspruch 3 gegen den menschlichen Gewebsplasminogen-Aktivator oder ein äquivalentes Fragment davon ist.

13. Reagenssystem nach Anspruch 12, dadurch **gekennzeichnet,** daß der erste Antikörper der monoclonale Antikörper JTI-3 oder JTI-4 gegen den menschlichen Plasminogen-Aktivator-Inhibitor 1 oder ein äquivalentes Fragment davon ist, und der zweite Antikörper der monoclonale Antikörper JTA-1 gegen menschlichen Gewebsplasminogen-Aktivator oder ein äquivalentes Fragment davon ist.

14. Reagenssystem nach Anspruch 12 oder 13, dadurch **gekennzeichnet,** daß der monoclonale Antikörper gegen den menschlichen Plasminogen-Aktivator-Inhibitor 1 der monoclonale Antikörper JTI-4 ist.

15. Reagenssystem nach einem der Ansprüche 12 bis 14, dadurch **gekennzeichnet,** daß der unlösliche feste Träger ein Plastikbehälter, eine Plastikkugel, ein Plastikstab, eine Latexkugel, ein Glasstab oder ein Metallteilchen ist.

16. Reagenssystem nach einem der Ansprüche 12 bis 15, dadurch **gekennzeichnet,** daß der markierte zweite Antikörper ein mit einem Enzym, einem Radioisotop, einer lumineszenten Substanz oder einer fluoreszenten Substanz markierter Antikörper ist.

17. Reagenssystem nach Anspruch 12, dadurch **gekennzeichnet,** daß der markierte zweite Antikörper ein Fab'-Fragment eines monoclonalen Antikörpers ist.

**18.** Reagenssystem nach Anspruch 12, dadurch **gekennzeichnet,** daß der markierte zweite Antikörper ein Fab'-Fragment eines monoclonalen Antikörpers, der mit einem Enzym markiert ist, ist.

**19.** Reagenssystem nach Anspruch 18, dadurch **gekennzeichnet,** daß der markierte zweite Antikörper ein mehrfach markierter Antikörper, umfassend ein Fab'-Fragment eines Antikörpers und mindestens durchschnittlich 1,5 Moleküle pro Molekül des Fab'-Fragments eines über das Schwefelatom des Fab'-Fragments daran gebundenen Enzyms, ist.

**20.** Reagenssystem nach Anspruch 12, dadurch **gekennzeichnet,** daß der unlösliche feste Träger eine Polystyrolkugel, deren Oberfläche glatt gemacht wurde, ist.

**21.** Kit zum immunologischen Assay auf einen menschlichen Plasminogen-Aktivator-Inhibitor 1-menschlichen Gewebsplasminogen-Aktivator-Komplex in einer menschlichen Testprobe, umfassend eine Kombination aus
(i) einem Reagenssystem nach Anspruch 12,
(ii) einem Lösungsmittel,
(iii) einem Waschmittel, und
(iv) sofern ein mit einem Enzym markierter Antikörper verwendet wird, ein Substrat zum Messen der Enzymaktivität und ein Mittel zum Stoppen der Reaktion,
wobei einer der Antikörper der monoclonale Antikörper JTI-3 nach Anspruch 1 oder JTI-4 nach Anspruch 2 gegen den menschlichen Plasminogen-Aktivator-Inhibitor 1 oder ein äquivalentes Fragment davon ist, und der andere der monoclonale Antikörper JTA-1 nach Anspruch 3 gegen menschlichen Gewebsplasminogen-Aktivator ist.

**22.** Verfahren zur Diagnose des Thrombosezustands, dadurch **gekennzeichnet,** daß man einen menschlichen Plasminogen-Aktivator-Inhibitor 1-menschlichen Gewebsplasminogen-Aktivator-Komplex in einer menschlichen Testprobe nach dem Verfahren nach Anspruch 4 bestimmt und den bestimmten Wert analysiert.

**Revendications**

**1.** Anticorps monoclonal JTI-3 contre l'inhibiteur d'activateur du plasminogène humain 1 qui a été produit par l'hybridome JTI-3 déposé sous le numéro de dépôt BP-2317 au Fermentation Research Institute, Japon.

**2.** Anticorps monoclonal JTI-4 contre l'inhibiteur d'activateur du plasminogène humain 1 qui a été produit par l'hybridome JTI-4 déposé sous le numéro de dépôt BP-2318 au Fermentation Research Institute, Japon.

**3.** Anticorps monoclonal JTA-1 contre l'activateur du plasminogène humain qui a été produit par l'hybridome JTA-1 déposé sous le numéro de dépôt BP-2316 au Fermentation Research Institute, Japon.

**4.** Procédé de dosage immunologique d'un complexe inhibiteur d'activateur du plasminogène humain 1-activateur du plasminogène de tissu humain dans un échantillon humain à doser qui comprend les opérations consistant :
(i) à amener l'échantillon humain à doser en contact avec un premier anticorps lié à un support solide insoluble, ensuite à laver le support et à l'amener en contact avec un deuxième anticorps marqué, ou
(ii) à amener le premier anticorps lié au support solide insoluble, le deuxième anticorps marqué et l'échantillon humain à doser en contact les uns avec les autres dans le même système de dosage,
dans lequel l'un des premier et second anticorps est l'anticorps monoclonal JTI-3 de la revendication 1 ou JTI-4 de la revendication 2 contre l'inhibiteur d'activateur du plasminogène humain 1 ou un fragment équivalent de ceux-ci, et l'autre est l'anticorps monoclonal JTA-1 de la revendication 3 contre l'activateur du plasminogène de tissu humain ou un fragment équivalent de celui-ci.

**5.** Procédé de dosage immunologique d'un complexe inhibiteur d'activateur du plasminogène humain 1___activateur plasminogène de tissu humain dans un échantillon humain à doser qui comprend les étapes consistant :

(i) à amener l'échantillon humain à doser humain en contact avec un premier anticorps lié à un support solide insoluble, ensuite à laver le support et à l'amener en contact avec un second anticorps marqué, ou

(ii) à amener le premier anticorps lié au support solide insoluble, le deuxième anticorps marqué et l'échantillon humain à doser en contact les uns avec les autres dans le même système de dosage,

dans lequel l'un des anticorps est l'anticorps monoclonal JTI-3 de la revendication 1 ou JTI-4 de la revendication 2 contre l'inhibiteur d'activateur du plasminogène humain 1, et l'autre est l'anticorps monoclonal JTA-1 de la revendication 3 contre l'activateur du plasminogène de tissu humain, et le second anticorps marqué est un fragment Fab' marqué d'un anticorps monoclonal.

6. Procédé selon la revendication 5, dans lequel le second anticorps marqué est un fragment Fab' d'un anticorps monoclonal marqué avec une enzyme.

7. Procédé selon la revendication 5, dans lequel le deuxième anticorps marqué est un anticorps multimarqué comprenant un fragment Fab d'un anticorps et au moins 1,5 molécules en moyenne, par molécule du fragment Fab', d'une enzyme liée à celui-ci par l'intermédiaire des atomes de soufre du fragment Fab'.

8. Procédé selon la revendication 5, dans lequel le support solide insoluble est une bille de polystyrène dont la surface est rendue lisse.

9. Procédé selon la revendication 5, dans lequel la réaction immunologique est réalisée en présence d'un inhibiteur d'adsorption non spécifique.

10. Procédé selon la revendication 9, dans lequel l'inhibiteur d'adsorption non spécifique est au moins une protéine ayant un poids moléculaires d'environ 16 000 à environ 50 000 et un point isoélectrique de 1,0 à 5,0, et la concentration finale de la protéine dans la solution de réaction immunologique est 0,02 à 0,9% en poids.

11. Procédé selon la revendication 10, dans lequel la réaction immunologique est réalisée en présence de lait écrémé, et la concentration finale du lait écrémé dans la solution de réaction immunologique est ajustée à 0,002 à 0,8% en poids.

12. Système de réactifs pour un dosage immunologique d'un complexe inhibiteur d'activateur du plasminogène humain 1___activateur du plasminogène de tissu humain dans un échantillon humain à doser en utilisant un premier anticorps lié à un support solide insoluble et un deuxième anticorps marqué, l'un des anticorps étant l'anticorps monoclonal JTI-3 de la revendication 1 ou JTI-4 de la revendication 2 contre l'inhibiteur d'activateur du plasminogène humain 1 ou un fragment équivalent de celui-ci et l'autre étant l'anticorps monoclonal JTA-1 de la revendication 3 contre l'activateur du plasminogène de tissu humain ou un fragment équivalent de celui-ci.

13. Système de réactifs selon la revendication 12, dans lequel le premier anticorps est l'anticorps monoclonal JTI-3 ou JTI-4 contre l'inhibiteur d'activateur du plasminogène humain 1 ou un fragment équivalent de celui-ci, et le deuxième anticorps est l'anticorps monoclonal JTA-1 contre l'activateur du plasminogène de tissu humain ou un fragment équivalent de celui-ci.

14. Système de réactifs selon la revendication 12 ou 13, dans lequel l'anticorps monoclonal contre l'inhibiteur d'activateur du plasminogène humain 1 est l'anticorps monoclonal JTI-4.

15. Système de réactifs selon l'une quelconque des revendications 12 à 14, dans lequel le support solide insoluble est un réceptacle de matière plastique, une bille de matière plastique, un baton de matière plastique, une bille de latex, une bille de verre ou une particule métallique.

16. Système de réactifs selon l'une quelconque des revendications 12 à 15 dans lequel le second anticorps marqué est un anticorps marqué avec une enzyme, un radioisotope, une substance luminescente ou une substance fluorescente.

33

**17.** Système de réactifs selon la revendication 12, dans lequel dans le second anticorps marqué, l'anticorps est un fragment Fab' d'un anticorps monoclonal.

**18.** Système de réactifs selon la revendication 12, dans lequel le second anticorps marqué est un fragment Fab' d'un anticorps monoclonal, qui est marqué avec une enzyme.

**19.** Système de réactifs selon la revendication 18, dans lequel le second anticorps marqué est un anticorps multimarqué comprenant un fragment Fab' d'un anticorps et au moins 1,5 molécules en moyenne, par molécule du fragment Fab', d'une enzyme liée à celui-ci par l'intermédiaire des atomes de soufre du fragment Fab'.

**20.** Système de réactifs selon la revendication 12, dans lequel le support solide insoluble est une bille de polystyrène dont la surface est rendue lisse.

**21.** Trousse pour le dosage immunologique d'un complexe inhibiteur d'activateur du plasminogène humain 1___activateur du plasminogène de tissu humain dans un échantillon humain à doser, comprenant une combinaison de
(i) un système de réactifs selon la revendication 12,
(ii) un agent de dissolution,
(iii) un agent de lavage, et
(iv) quand un anticorps marqué avec une enzyme est utilisé, un substrat pour mesurer l'activité enzymatique et un agent d'arrêt de réaction,
l'un des anticorps étant l'anticorps monoclonal JTI-3 de la revendication 1 ou JTI-4 de la revendication 2 contre l'inhibiteur d'activateur du plasminogène humain 1 ou un fragment équivalent de celui-ci et l'autre étant l'anticorps monoclonal JTA-1 de la revendication 3 contre l'activateur du plasminogène de tissu humain.

**22.** Procédé de diagnostic de l'état de thrombose, qui comprend la détermination d'un complexe inhibiteur d'activateur du plasminogène humain 1___activateur du plasminogène de tissu humain dans un échantillon humain à doser humain à l'aide du procédé selon la revendication 4, et d'analyse de la valeur déterminée.

*Fig. 1*

IMMUNOBLOTTING BY JTI-2

PAI·I—tPA
COMPLEX

PAI·I

MOLECULAR
WEIGHT

— 92. 5K

— 66. 2K

— 45. OK

— 31. OK

— 21. 5K

*Fig. 2*

○——○ JTI-1
●——● JTI-2
□——□ JTI-3
■——■ JTI-4

ABSORBANCE

1.5

1.0

0.5

0

0    10    20    30    40

CONCENTRATION OF
PAI·I—tPA COMPLEX ( ng/ml )

Fig. 3-1

Fig. 3-2

FIRST ANTIBODY: JTA-3

FIRST ANTIBODY: JTA-4

JTI-1
JTI-2
JTI-3
JTI-4

ABSORBANCE

CONCENTRATION OF
PAI·I−tPA COMPLEX (ng/ml)

CONCENTRATION OF
PAI·I−tPA COMPLEX (ng/ml)

EP 0 339 302 B1

Fig. 4

Fig. 6

Fig. 5

FIRST ANTIBODY: JTI-4

FIRST ANTIBODY: JTI-3

○——○ LATENT OR
       QUISCENT PAI·I

●——● ACTIVATED PAI·I

CHANGE IN ABSORBANCE PER UNIT TIME

CONCENTRATION OF FREE
PAI·I          (ng/ml)

CHANGE IN ABSORBANCE PER UNIT TIME

CONCENTRATION OF FREE
PAI·I          (ng/ml)

EP 0 339 302 B1

Fig. 7-1

DETERMINATION OF THE TOTAL PAI·I ANTIGEN

*Fig. 7-2*

FIRST ANTIBODY : JTI-3
SECOND ANTIBODY : JTI-4

A
B
C
D
E

ABSORBANCE (495 nm)

CONCENTRATION OF
PAI·I COMPLEX (ng/ml)

*Fig. 7-3*

FIRST ANTIBODY : JTI-4
SECOND ANTIBODY : JTI-3

ABSORBANCE (495 nm)

CONCENTRATION OF
PAI·I COMPLEX (ng/ml)

EP 0 339 302 B1

# Fig. 8

INHIBITION BY ANTI-PAI.1 MONOCLONAL
ANTIBODY OF THE FORMATION OF PAI.1-tPA
COMPLEX IN VASCULAR WALL ENDOTHELIAL
CELL

1  2  3  4  5

— PAI·1-tPA COMPLEX

— tPA

# Fig. 11

SILVER STAINING AND IMMUNOBLOTTING
OF PURIFIED PAI

A  B  C  D  E  F  G

— PAI·1

Fig. 9

DEGREE OF FIBRIN DISSOLUTION MEASURED WHEN 6 ng/ml OF
SDS-ACTIVATED PAI.1 AND JTI-3 OF VARIOUS CONCENTRATIONS
WERE ADDED TO 0.0025 U/ml tPA

DEGREE OF FIBRIN DISSOLUTION (% LYSIS)

SEQUENTIALLY

SIMULTANEOUSLY

MOLAR RATIO    IgG / PAI·1

EP 0 339 302 B1

Fig. 10

## Fig. 12

PROTEIN STAINED PATTERN OF THE PAI.1-tPA
COMPLEX

A   B   C   D   E   F

← PAI·1-tPA COMPLEX

## Fig. 14

OD₄₅₀

Fab'-(HRP)₂ 0.6 μg/ml
1.520 (3.82)

Fab'-HRP 0.6 μg/ml
0.398 (1.0)

1.5

1.0

0.5

0

6.25    12.5         25

CONCENTRATION OF THE PAI·1-tPA COMPLEX  (ng/ml)

45

## Fig. 13

## Fig. 15

## Fig. 16

## Fig. 17

CONCENTRATION OF THE
PAI.1-tPA COMPLEX (ng/ml)

*Fig. 18*

CONCENTRATION OF PAI.1-tPA COMPLEX